# FASCICULE DE BREVET EUROPEEN

(11) **EP 0 858 459 B1**
(45) Date de publication et mention de la délivrance du brevet: **04.09.2002**
(21) Numéro de dépôt: 96934921.6
(22) Date de dépôt: 16.10.1996
(51) Int. Cl.: C07D 405/06, C07D 405/14, C07D 409/14

(54) **NOUVEAUX DERIVES DE PYRAZOLES ACIDES, LEUR PROCEDE DE PREPARATION, LEUR APPLICATION A TITRE DE MEDICAMENTS, LEUR NOUVELLE UTILISATION ET LES COMPOSITIONS PHARMACEUTIQUES LES RENFERMANT**
NEUE SÄUREDERIVATE DES PYRAZOLS, VERFAHREN ZU IHRER HERSTELLUNG, IHRE ANWENDUNG ALS MEDIKAMENTE, IHRE NEUE ANWENDUNG UND DIE SIE ENTHALTENDE PHARMAZEUTISCHE ZUSAMMENSETZUNGEN
NOVEL ACID PYRAZOLE DERIVATIVES, PREPARATION METHOD THEREFOR, USE THEREOF AS DRUGS, NOVEL USE THEREFOR, AND PHARMACEUTICAL COMPOSITIONS CONTAINING SUCH DERIVATIVES

(30) Priorité: 20.10.1995 FR 9512330
(43) Date de publication de la demande: 19.08.1998
(73) Titulaire: Aventis Pharma S.A., 92160 Antony (FR)
(72) Inventeur: DIDIERLAURENT, Stanislas, F-77400 Lagny-sur-Marne (FR); FORTIN, Michel, F-75018 Paris (FR); ZHANG, Jidong, F-75013 Paris (FR)
(86) Numéro de dépôt international: FR9601615
(87) Numéro de publication internationale: WO97015570

(56) Documents cités:
- EP-A- 0 449 699
- WO-A-94/02474
- WO-A-96/12706

## Description

La présente invention concerne de nouveaux dérivés de pyrazoles acides, leur procédé de préparation, les nouveaux intermédiaires obtenus, leur application à titre de médicaments, les compositions pharmaceutiques les renfermant et la nouvelle utilisation de tels dérivés de pyrazoles acides.

La présente invention a pour objet les produits de formule (I) : 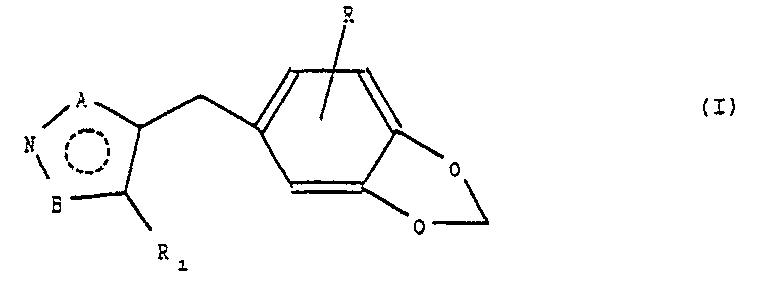 dans laquelle
A représente soit un atome d'azote substitué par un radical alkyle linéaire ou ramifié, renfermant au plus 4 atomes de carbone,
   soit un radical méthine substitué par un radical phényle, thiényle ou pyridyle,
B représente soit un atome d'azote substitué par un radical alkyle renfermant au plus 4 atomes de carbone lui-même substitué par un radical cyclohexyle, lui-même éventuellement substitué par un radical carboxy ou par un radical carboxyméthoxyméthyle,
   soit un radical méthine substitué par un radical alkylthio, renfermant au plus 6 atomes de carbone ou par un radical cyclohexylthio, ces radicaux étant éventuellement substitués par un radical carboxy ou par un radical phényle lui-même substitué par un radical alcoxy renfermant au plus 4 atomes de carbone, étant entendu que l'un de A et B représente un atome d'azote et l'autre de A et B représente un radical méthine, ces radicaux étant substitués comme indiqué ci-dessus,
R₁ représente un radical carboxy libre ou estérifié par un radical alkyle linéaire ou ramifié, renfermant au plus 4 atomes de carbone,
R représente un atome d'halogène,
   ainsi que les produits suivants :
   - acide 1-butyl 3-((4-(carboxyméthyl) cyclohexyl) thio) 5-((6(chloro 1,3-benzodioxol-5-yl) méthyl) 1H-pyrazole 4-carboxylique,
   - acide 3-((4-(carboxyméthyl) cyclohexyl) thio) 5-((6-chloro 1,3-benzodioxol-5-yl) méthyl) 1-propyl 1H-pyrazole 4-carboxylique,
   - acide 1-((bicyclo(2.2.2)oct-2-yl) méthyl) 4-((6-chloro 1,3-benzodioxol-5-yl) méthyl) 3-phényl 1H-pyrazole 5-carboxylique,
   - acide cis/trans 1-((4-carboxyméthyl)cyclohexyl)méthyl) 4-((6-chloro 1,3-benzodioxol-5-yl)méthyl) 3-(2-thiényl) 1H-pyrazole 5-carboxylique,
   - acide 1-((2-(carboxyméthoxy) phényl) méthyl) 4-((6-chloro 1,3-benzodioxol-5-yl) méthyl) 3-(2-thiényl) 1H-pyrazole 5-carboxylique,
   lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) ou dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés,
- le terme radical alkylthio désigne les radicaux dans lesquels le radical alkyle est tel que défini ci-dessus comme par exemple dans méthylthio, éthylthio, propylthio, isopropylthio, butylthio, sec-butylthio, tert-butylthio, isopentylthio, isohexylthio, mais aussi heptylthio, octylthio, nonylthio ou décylthio ainsi que leurs isomères de position linéaires ou ramifiés,
- le radical cycloalkylthio désigne notamment les radicaux cyclopentylthio et cyclohexylthio.
   Parmi les radicaux alcoxyalkyle on peut citer par exemple les radicaux méthoxyméthyle, éthoxyméthyle, méthoxyéthyle, éthoxypropyle, propyloxyméthyle, propyloxypropyle ou encore propyloxyéthyle.
   Les radicaux alkyle et alcoxyalkyle tels que définis ci-dessus peuvent être substitués par un radical carboxy libre, salifié ou estérifié pour donner les radicaux correspondants carboxyalkyle et carboxyalcoxyalkyle,
- le terme atome d'halogène désigne de préférence l'atome de chlore, mais peut aussi représenter un atome de fluor, de brome ou d'iode,

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.
   On préfère les sels de sodium ou de potassium.
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

On peut citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut citer plus particulièrement les sels formés avec les acides chlorhydrique ou méthanesulfonique par exemple.

On peut rappeler que la stéréoisomérie peut être définie dans son sens large comme l'isomérie de composés ayant mêmes formules développées, mais dont les différents groupes sont disposés différemment dans l'espace, tels que notamment dans les formes bateau et chaise du cyclohexane et des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a plus particulièrement pour objet les produits de formule (I) telle que définie ci-dessus, dont les noms suivent :
- acide 4-((6-chloro-1,3-benzodioxol-5-yl) méthyl)-1-(cyclohexylméthyl)-3-phényl-1H-pyrazole-5-carboxylique,
- acide 1-butyl-5-((6-chloro-1,3-benzodioxol-5-yl) méthyl)-3-((5-carboxypentyl) thio)-1H-pyrazole-4-carboxylique,
- acide 1-((3-carboxycyclohexyl) méthyl)-4-((6-chloro-1,3-benzodioxol-5-yl) méthyl)-3-phényl-1H-pyrazole-5-carboxylique,
- acide 4-((6-chloro-1,3-benzodioxol-5-yl) méthyl)-1-cyclohexylméthyl)-3-(2-thiényl)-1H-pyrazole-5-carboxylique,
- acide 4-((6-chloro-1,3-benzodioxol-5-yl) méthyl)-1-(cyclohexylméthyl)-3-(2-pyridinyl)-1H-pyrazole-5-carboxylique,
- acide 4-((6-chloro-1,3-benzodioxol-5-yl) méthyl)-1-(cyclohexylméthyl)-3-(3-thiényl)-1H-pyrazole-5-carboxylique,
- acide 1-butyl 3-((4-(carboxyméthyl) cyclohexyl) thio) 5-((6(chloro 1,3-benzodioxol-5-yl) méthyl) 1H-pyrazole 4-carboxylique,
- acide 3-((4-(carboxyméthyl) cyclohexyl) thio) 5-((6-chloro 1,3-benzodioxol-5-yl) méthyl) 1-propyl 1H-pyrazole 4-carboxylique,
- acide 1-((bicyclo(2.2.2)oct-2-yl) méthyl) 4-((6-chloro 1,3-benzodioxol-5-yl) méthyl) 3-phényl 1H-pyrazole 5-carboxylique,
- acide 1-((4-carboxycyclohexyl) méthyl) 4-((6-chloro 1,3-benzodioxol-5-yl) méthyl) 3-(2-thiényl) 1H-pyrazole 5-carboxylique,
- acide cis/trans 1-((4-(carboxyméthyl) cyclohexyl) méthyl) 4-((6-chloro 1,3-benzodioxol-5-yl) méthyl) 3-(2-thiényl) 1H-pyrazole 5-carboxylique,
- acide 1-((2-(carboxyméthoxy) phényl) méthyl) 4-((6-chloro 1,3-benzodioxol-5-yl) méthyl) 3-(2-thiényl) 1H-pyrazole 5-carboxylique,
- acide 1-((3-carboxycyclohexyl) méthyl) 4-((6-chloro 1,3-benzodioxol-5-yl) méthyl) 3-(2-thiényl) 1H-pyrazole 5-carboxylique.

La présente invention a encore pour objet le procédé de préparation des produits de formule (I) telle que définie ci-dessus, caractérisé en ce que :
ou bien l'on fait agir un produit de formule (II) :

ZCA'-CO-CH₂-CO-CO₂alk (II)

dans laquelle ZCA' représente un radical phényle, thiényle ou pyridyle, et alk représente un radical alkyle linéaire ou ramifié, renfermant au plus 4 atomes de carbone, avec le produit de formule (III) : 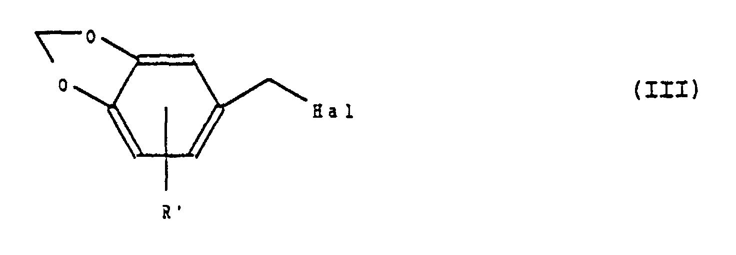 dans laquelle R' a la signification indiquée à la revendication 1 pour R, et Hal représente un atome d'halogène, pour obtenir le produit de formule (IV) : 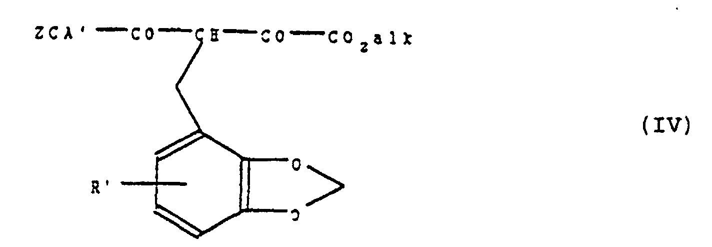 dans laquelle R', ZCA' et alk ont les significations indiquées ci-dessus, que l'on fait réagir avec l'hydrazine NH₂-NH₂ pour obtenir un produit de formule (V) : 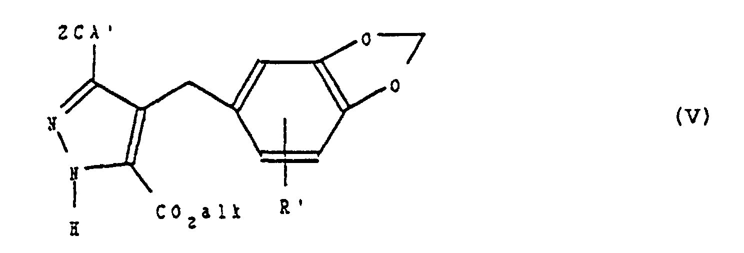 dans laquelle R', ZCA' et alk ont les significations indiquées ci-dessus,
que l'on fait réagir avec le composé de formule (VI) :

ZNB'-W (VI)

dans laquelle ZNB' représente un radical alkyle renfermant au plus 4 atomes de carbone lui-même substitué par un radical cyclohexyle, lui-même éventuellement substitué par un radical carboxy ou par un radical carboxyméthoxyméthyle, dans lequel les éventuelles fonctions réactives sont éventuellement protégées et W représente un atome d'halogène ou un radical tosyle, pour obtenir le produit de formule (I₁) 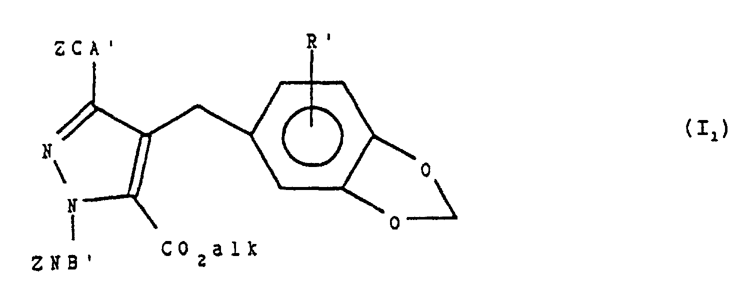 ou bien l'on fait agir un produit de formule (VII) : 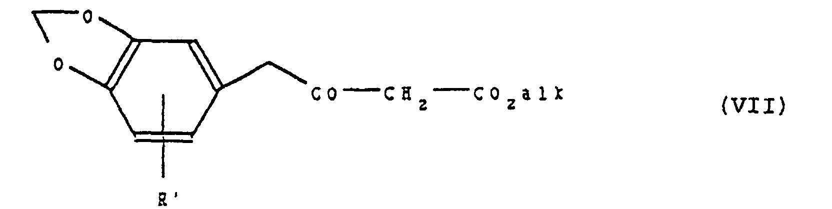 dans laquelle R' et alk ont les significations indiquées ci-dessus,
avec du disulfure de carbone et un produit de formule (VIII) :

R_{d}-Hal (VIII)

dans laquelle R_{d} représente un radical alkyle renfermant au plus 6 atomes de carbone éventuellement substitué par un radical carboxy ou par un radical phényle lui-même substitué par un radical alcoxy renfermant au plus 4 atomes de carbone, et Hal représente un atome d'halogène,
pour obtenir le produit de formule (IX) : 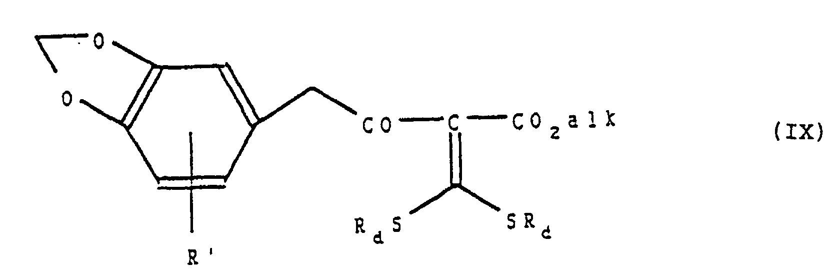 dans laquelle R', alk et R_{d} ont les significations indiquées ci-dessus et S représente un atome de soufre, que l'on traite avec un produit de formule (X) :

ZNA'-NH-NH₂ (X)

dans laquelle ZNA' représente un radical alkyle linéaire ou ramifié, renfermant au plus 4 atomes de carbone,pour obtenir le produit de formule (I₂) : 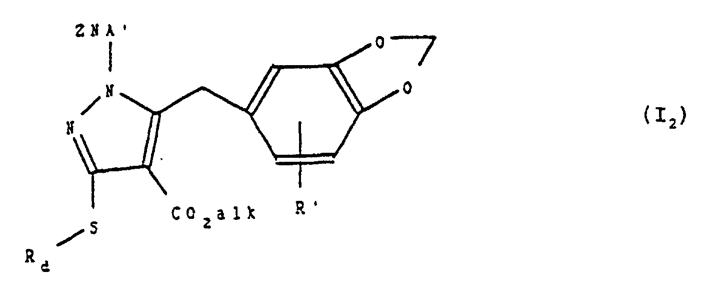 dans laquelle ZNA', alk, R_{d} et S ont les significations indiquées ci-dessus,
ou bien l'on fait agir un produit de formule (XI) : 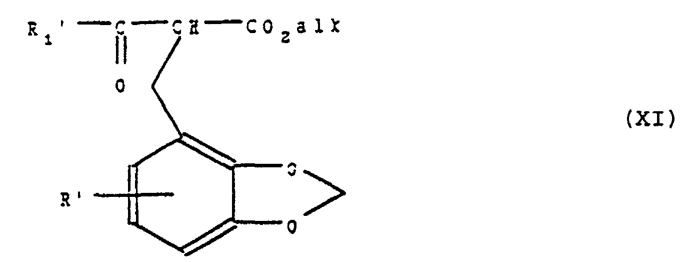 dans laquelle R' et alk ont les significations indiquées ci-dessus et R'₁ a la signification indiquée à la revendication 1 pour R₁, dans laquelle les éventuelles fonctions réactives sont éventuellement protégées, avec le dérivé de l'hydrazine suivant :

N≡C-(CH₂)₂-NH-NH₂

pour obtenir un produit de formule (XII) : 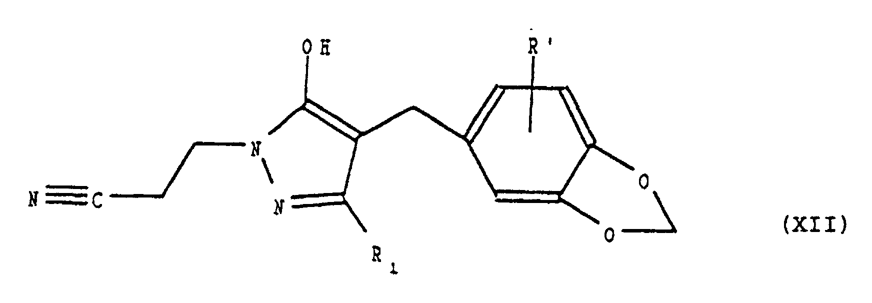 dans laquelle R' et R'₁ ont les significations indiquées ci-dessus, que l'on transforme en produit de formule (XIII) : 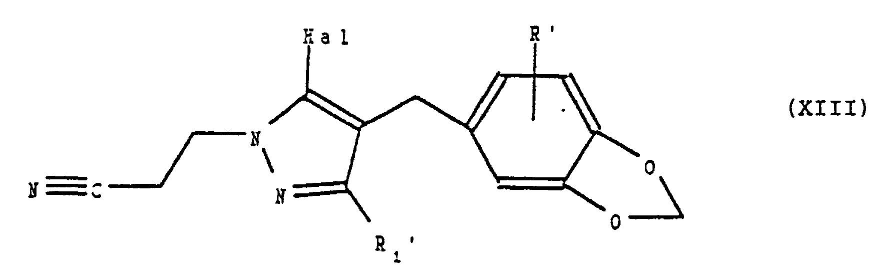 dans laquelle Hal, R' et R'₁ ont les significations indiquées ci-dessus, que l'on soumet à l'action d'une base puis au composé de formule (VI) telle que définie ci-dessus,
pour obtenir le produit de formule (XIV) : 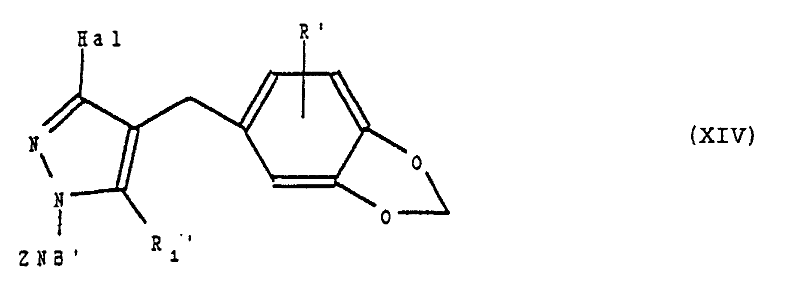 dans laquelle R', R'₁, ZNB' et Hal ont les significations indiquées ci-dessus,
que l'on soumet à une réaction de substitution par un dérivé de l'acide boronique ou par un organo metallique, sur l'atome d'halogène pour obtenir le produit de formule (I₃) : 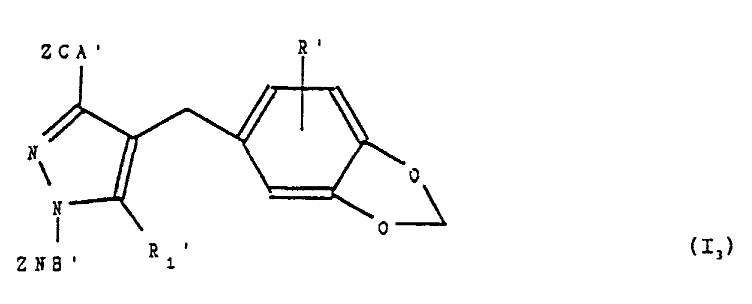 dans laquelle ZCA', ZNB', R' et R'₁ ont les significations indiquées ci-dessus,
ou bien l'on fait agir le produit de formule (VII) telle que définie ci-dessus, avec un produit de formule (XV) : 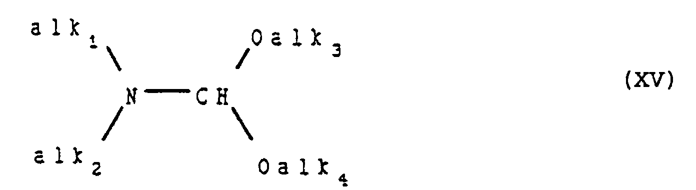 dans laquelle alk₁, alk₂, alk₃ et alk₄, identiques ou différents, représentent un radical alkyle linéaire ou ramifié, renfermant au plus 4 atomes de carbone, pour obtenir un produit de formule (XVI) : 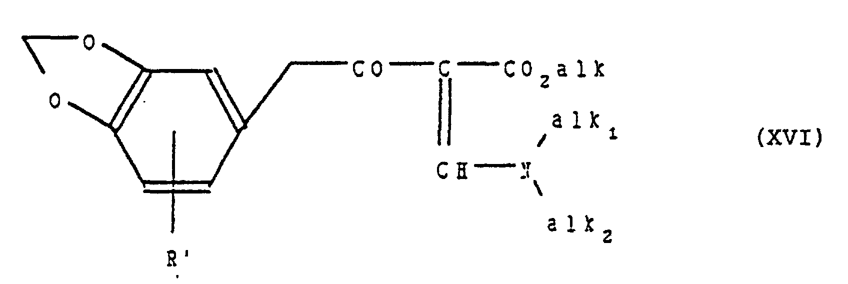 dans laquelle R', alk, alk₁ et alk₂ ont les significations indiquées ci-dessus, que l'on traite par l'hydrazine, pour obtenir le produit de formule (XVII) : 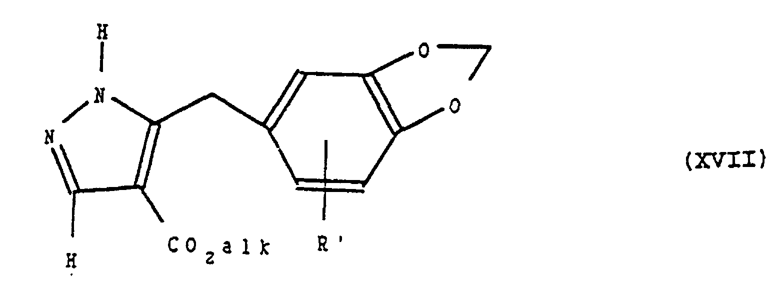 dans laquelle R' et alk ont les significations indiquées ci-dessus, que l'on traite par un agent d'halogénation pour obtenir un produit de formule (XVIII) : 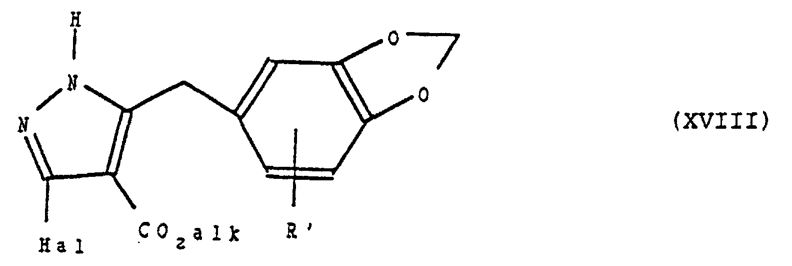 dans laquelle R', Hal et alk ont les significations indiquées ci-dessus, que l'on traite par le produit de formule (XIX) :

ZNA'-Hal (XIX)

dans laquelle ZNA' et Hal ont les significations indiquées ci-dessus, pour obtenir le produit de formule (XX) : 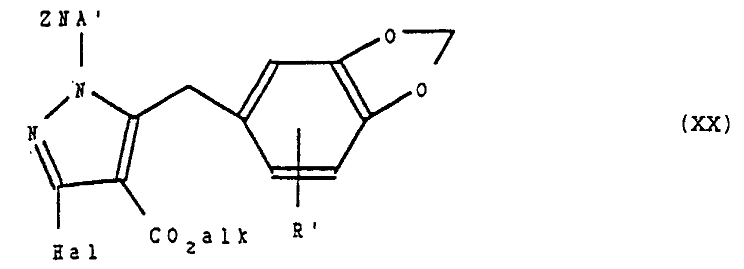 dans laquelle R' et alk ont les significations indiquées ci-dessus, sur lesquelles on fait agir un produit de formule (XXI) :

ZCB'-M (XXI)

dans laquelle ZCB' représente un radical alkylthio, renfermant au plus 6 atomes de carbone ou un radical cyclohexylthio, ces radicaux étant éventuellement substitués par un radical carboxy ou par un radical phényle lui-même substitué par un radical alcoxy renfermant au plus 4 atomes de carbone, et M représente un métal, pour obtenir le produit de formule (I₄) : 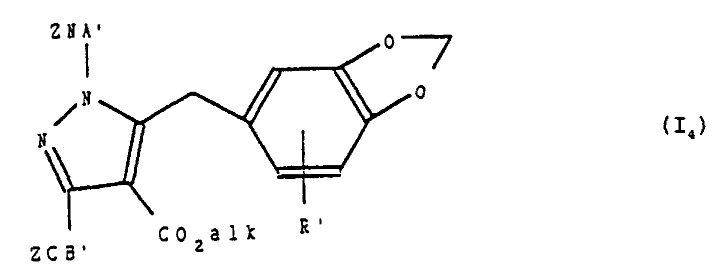 dans laquelle R', alk, ZNA' et ZCB' ont les significations indiquées ci-dessus,
produits de formules (I₁), (I₂), (I₃) et (I₄) telles que définies ci-dessus, que l'on peut soumettre, si désiré et si nécessaire, à l'une ou plusieurs des réactions de transformations suivantes, dans un ordre quelconque :
a) une réaction de saponification de fonction ester en fonction acide,
f) une réaction de transformation de la fonction ester en radical hydroxyalkyle ou alcoxyalkyle puis en radical alkyle,
g) une réaction d'élimination des groupements protecteurs que peuvent porter les fonctions réactives protégées,
h) une réaction de salification par un acide minéral ou organique ou par une base pour obtenir le sel correspondant,
i) une réaction de dédoublement des formes racémiques en produits dédoublés,
   lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut être réalisé de la façon suivante :
l'action des produits de formule (III) sur les produits de formule (II), pour obtenir les produits de formule (IV) est effectuée en présence d'une base forte ou d'un carbonate : on peut opérer par exemple en présence d'hydrure ou d'éthylate de sodium dans un solvant aprotique tel que le toluène ou encore en présence de carbonate de sodium ou de potassium par exemple dans le diméthylformamide.

Les conditions de cette réaction sont par exemple décrites dans J. Org. Chem. Vol. 39, N° 22, (1974), p. 3271.

Dans le composé de formule (III), Hal représente de préférence un atome de chlore, de brome ou d'iode.

On peut opérer en présence d'un catalyseur tel que l'aliquat 336 et d'iodure de potassium notamment lorsque Hal représente un atome de chlore.

L'action de l'hydrazine ou de ses dérivés, notamment des produits de formule (X), sur les produits de formule (IV), (IX), (XI) ou (XVI) pour obtenir respectivement les produits de formule (V), (I₂), (XII) ou (XVII) peut être effectuée en présence d'un acide tel que l'acide acétique au reflux ou encore dans un alcool tel que le méthanol ou l'éthanol.

Lorsque les dérivés de l'hydrazine sont sous forme d'un sel d'hydrazine qui peut être par exemple le chlorhydrate, on opère de préférence en présence d'une base telle que la soude ou l'ammoniaque pour obtenir d'abord la base libre.

Le radical OH du produit de formule (XII) est transformé en un atome d'halogène par exemple par action d'oxychlorure ou -bromure de phosphore pour donner le produit de formule (XIII).

La réaction du produit de formule (XIII) ainsi obtenu avec le composé de formule (VI) pour obtenir le produit de formule (XIV) peut être réalisée par action préalable d'une base telle que par exemple l'hydrure de sodium dans un solvant tel que le diméthylformamide puis par action du produit de formule (VI) dans lequel l'atome d'halogène peut notamment être un atome de brome.

La réaction du composé de formule (XIV) ainsi obtenu avec un dérivé de l'acide boronique ou un organométallique pour obtenir un produit de formule (I₃) peut être réalisée en présence d'un catalyseur tel que le palladium dans un solvant, tel que par exemple le diméthylformamide ou le toluène.

Une illustration de telles réactions est donnée dans la préparation des exemples 8, 9 et 10 décrite ci-après.

La réaction du produit de formule (VII) avec le produit de formule (VIII) et du disulfure de carbone pour obtenir le produit de formule (IX) peut être réalisée en présence de fluorure de potassium sur alumine.

La réaction du produit de formule (VII) avec le produit de formule (XV) pour obtenir le produit de formule (XVI) peut être réalisée par simple mise en présence des deux produits de préférence à chaud.

L'halogénation du produit de formule (XVII) en produit de formule (XVIII) peut être réalisée dans les conditions usuelles par un agent d'halogénation tel que par exemple le brome dans l'acide acétique.

La réaction du produit de formule (XX) avec le produit de formule (XXI) pour obtenir le produit de formule (I₄) peut être réalisée dans les conditions usuelles d'un organo métallique tel que par exemple un organo-zincique, -stannane ou un acide organoboronique, en présence d'un catalyseur tel que par exemple le palladium tétrakis.

Dans le composé de formule (XXI), M représente de préférence le zinc, l'étain ou un atome de bore : comme exemple d'un tel composé, on peut citer le zincique de iodo benzyle.

Dans les composés de formules (VI) et (XIX), Hal représente un atome d'halogène mais pourrait aussi être un pseudo-halogène tel qu'un tosyle ou un mésyle.

La réaction du produit de formule (V) avec le produit de formule (VI) pour obtenir un produit de formule (I₁) ou la réaction du produit de formule (XVIII) avec le produit de formule (XIX) pour obtenir le produit de formule (XX) peut être réalisée par exemple à chaud dans un solvant aprotique tel que le toluène ou le diméthylformamide, avec par exemple un halogénure d'alkyle tel que l'iodure de méthyle ou le bromure de cyclohexylméthyle ou encore avec le 1-[(3-tosylméthyl) cyclohexyl] carboxylate d'éthyle en présence d'une base telle que par exemple l'hydrure de sodium ou du carbonate de sodium ou de potassium.

Les produits de formules (I₁), (I₂), (I₃) et (I₄) constituent des produits de formule (I) et peuvent être transformés en d'autres produits de formule (I) en étant soumis à une où plusieurs des réactions a) à i) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁), (I₂), (I₃) et (I₄), telles que définies ci-dessus peuvent être soumis, si désiré ou si nécessaire, peuvent être réalisées, par exemple, comme indiqué ci-après :
a) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique,
f) la réaction de transformation de fonction ester telles que définies ci-dessus peut être effectuée dans les conditions usuelles connues de l'homme de métier,
g) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
h) Les produits décrits ci-dessus peuvent, si désiré, faire l'objet de réactions de salification par exemple par un acide minéral ou organique ou par une base minérale ou organique selon les méthodes usuelles connues de l'homme du métier.
i) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de formule (I) tels que définis ci-dessus ainsi que leurs sels d'addition avec les acides présentent d'intéressantes propriétés pharmacologiques.

Les produits de formule (I) tels que définis ci-dessus, sont doués de propriétés antagonistes pour les récepteurs à l'endothélin et sont ainsi notamment inhibiteurs des effets de l'endothéline, notamment des effets vaso-constricteurs et hypertenseurs induits par l'endothéline. On note en particulier un effet antiischémique, l'activité vasoconstrictrice de l'endothéline étant abolie.

Les produits de formule (I) sont également capables de s'opposer aux effets stimulants de l'endothéline au niveau de tous les types cellulaires, notamment les cellules musculaires lisses, les fibroblastes, les cellules neuronales et les cellules osseuses.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a plus particulièrement pour objet, à titre de médicaments, les produits décrits ci-après dans les exemples et notamment les produits de formule (I) dont les noms suivent :
- acide 4-((6-chloro-1,3-benzodioxol-5-yl) méthyl)-1-(cyclohexylméthyl)-3-phényl-1H-pyrazole-5-carboxylique,
- acide 1-butyl-5-((6-chloro-1,3-benzodioxol-5-yl) méthyl)-3-((5-carboxypentyl) thio)-1H-pyrazole-4-carboxylique,
- acide 1-((3-carboxycyclohexyl)-méthyl)-4-((6-chloro-1,3-benzodioxol-5-yl) méthyl)-3-phényl-1H-pyrazole-5-carboxylique,
- acide 4-((6-chloro-1,3-benzodioxol-5-yl) méthyl)-1-cyclohexylméthyl)-3-(2-thiényl)-1H-pyrazole-5-carboxylique,
- acide 4-((6-chloro-1,3-benzodioxol-5-yl) méthyl)-1-(cyclohexylméthyl)-3-(2-pyridinyl)-1H-pyrazole-5-carboxylique,
- acide 4-((6-chloro-1,3-benzodioxol-5-yl) méthyl)-1-(cyclohexylméthyl)-3-(3-thiényl)-1H-pyrazole-5-carboxylique,
- acide 1-butyl 3-((4-(carboxyméthyl) cyclohexyl) thio) 5-((6(chloro 1,3-benzodioxol-5-yl) méthyl) 1H-pyrazole 4-carboxylique,
- acide 3-((4-(carboxyméthyl) cyclohexyl) thio) 5-((6-chloro 1,3-benzodioxol-5-yl) méthyl) 1-propyl 1H-pyrazole 4-carboxylique,
- acide 1-((bicyclo(2.2.2)oct-2-yl) méthyl) 4-((6-chloro 1,3-benzodioxol-5-yl) méthyl) 3-phényl 1H-pyrazo 5-carboxylique,
- acide 1-((4-carboxycyclohexyl) méthyl) 4-((6-chloro 1,3-benzodioxol-5-yl) méthyl) 3-(2-thiényl) 1H-pyrazole 5-carboxylique,
- acide cis/trans 1-((4-(carboxyméthyl) cyclohexyl) méthyl) 4-((6-chloro 1,3-benzodioxol-5-yl) méthyl) 3-(2-thiényl) 1H-pyrazole 5-carboxylique,
- acide 1-((2-(carboxyméthoxy) phényl) méthyl) 4-((6-chloro 1,3-benzodioxol-5-yl) méthyl) 3-(2-thiényl) 1H-pyrazole 5-carboxylique,
- acide 1-((3-carboxycyclohexyl) méthyl) 4-((6-chloro 1,3-benzodioxol-5-yl) méthyl) 3-(2-thiényl) 1H-pyrazole 5-carboxylique,
ainsi que leurs sels d'addition avec les acides minéraux ou organiques ou avec les bases minérales et organiques pharmaceutiquement acceptables.

Les médicaments, objet de l'invention, trouvent, par exemple, leur emploi dans le traitement de tous les spasmes vasculaires, dans le traitement des suites d'une hémorragie cérébrale, dans le traitement des spasmes coronariens, des spasmes vasculaires périphériques ainsi que dans le traitement des insuffisances rénales. Ces médicaments peuvent également être utilisés dans le traitement de l'infarctus du myocarde, de l'insuffisance cardiaque congestive, dans la prévention des resténoses post-angioplastie, la prévention des fibroses cardiaques et vasculaires, dans le traitement de l'athérosclérose et de certaines formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hyperplasie prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Les produits de départ de formules (II), (III), (VI), (VII), (VIII), (X), (XI), (XV), (XIX) et (XXI) sont pour la plupart commerciaux ou peuvent être préparés à partir de produits commerciaux par les méthodes usuelles connues de l'homme du métier.

A titre d'exemple et de façon non exhaustive, on peut citer :
- parmi les produits de formule (II), le benzoyl pyruvate d'éthyle ou les produits correspondants dans lesquels le radical phényle est remplacé par un radical thiényle ou pyridyle,
- parmi les produits de formule (VII), on peut citer le dérivé d'acétoacétate d'éthyle dans lequel R' représente un atome d'hydrogène ou d'halogène,
- parmi les produits de formule (XI), on peut citer le composé dans lequel R'₁ représente un radical carboxy estérifié ou tétrazolyle,
- parmi les produits de formule (XIX), on peut citer le bromure de cyclohexylméthyle ou de carboxycyclohexylméthyle,
- parmi les produits de formulé (XV), on peut citer le diméthylacétal de diméthylformamide,
- parmi les produits de formule (III), on peut citer notamment le chlorure de 6-chloro pipéronyle produit commercial, le chlorure de pipéronyle, le chlorure de 3,4-dichlorobenzyle, ou encore le bromure de 3-chlorobenzyle,
- parmi les produits de formule (III) non commerciaux, le chlorure de 5-chloropipéranyle peut être préparé selon les méthodes usuelles connues de l'homme du métier,
- parmi les produits de formules (VIII) et (XIX), on peut citer le iodure de méthyle, de butyle ou de cycloalkyle ou encore pour le produit de formule (XIX) le chlorure de vinyle,
- parmi les produits de formules (X), on peut citer la méthyl-, butyl- ou butényl-hydrazine notamment sous forme de chlorhydrate préparées à partir de l'hydrazine de façon usuelle,
- parmi les produits de formule (XXI), on peut citer le butyl- ou cyclohexyl-magnésium.

Les exemples décrits ci-après dans la partie expérimentale donnent des illustrations, par ailleurs non exhaustives, de tels produits de départ.

La présente invention a enfin pour objet à titre de produits industriels nouveaux, les composés de formules (XII), (XIII) et (XIV), quand R'₁ est différent de carboxy, ainsi que les produits de formule (XX).

L'invention a ainsi particulièrement pour objet l'utilisation des produits de formule (I) telle que définie ci-dessus, pour la préparation de compositions pharmaceutiques destinées au traitement d'affections résultant d'une stimulation anormale des récepteurs de l'endothéline.

L'invention a plus particulièrement pour objet l'utilisation des produits de formule (I) telle que définie ci-dessus, pour la préparation de compositions pharmaceutiques destinées au traitement de l'hypertension induite par l'endothéline.

L'invention a tout particulièrement pour objet l'utilisation des produits de formule (I) telle que définie ci-dessus, pour la préparation de compositions pharmaceutiques destinées au traitement de tous les spasmes vasculaires et du traitement des suites d'une hémorragie cérébrale et des insuffisances rénales.

L'invention a également pour objet l'utilisation des produits de formule (I) telle que définie ci-dessus, pour la préparation de compositions pharmaceutiques destinées au traitement de l'infarctus du myocarde, à la prévention des resténoses post-angioplastie et à la prévention des fibroses cardiaques et vasculaires.

Les exemples suivants illustrent l'invention sans toutefois la limiter.

### EXEMPLE 1 : Acide 4-((6-chloro-1,3-benzodioxol-5-yl) méthyl)-1-(cyclohexylméthyl)-3-phényl-1H-pyrazole-5-carboxylique

### STADE 1 : β-benzoyl 6-chloro α-oxo-1,3-benzodioxol 5-butanoate d'éthyle

On introduit 12,7 g de benzoyl pyruvate d'éthyle (préparé comme indiqué dans la publication Tetrahedron Letter, 29, (1988), 3997-4000), 100 ml de diméthylformamide, 3 g d'éthylate de sodium, agite à température ambiante durant 15 minutes puis ajoute 11,81 g de chlorure de 6-chloropipéronyle et 8,30 g d'iodure de potassium. On agite à température ambiante durant 24 heures, verse dans 100 ml d'eau, extrait par 3 x 50 ml d'acétate d'éthyle, lave par 50 ml de chlorure de sodium et sèche. On obtient ainsi 22,8 g de produit attendu et l'utilise tel quel pour le stade suivant.

### STADE 2 : 4-((6-chloro 1,3-benzodioxol-5-yl) méthyl)-5-phényl 1H-pyrazole-3-carboxylate d'éthyle

On ajoute 2,8 ml d'hydrate d'hydrazine à 22,8 g du produit obtenu au stade 1 ci-dessus, dans 114 ml d'éthanol et chauffe 4 heures au reflux. On refroidit à température ambiante, essore, rince à l'éthanol et obtient 9,11 g de produit attendu. F = 204°C.

### STADE 3 : 4-((6-chloro-1,3-benzodioxol-5-yl) méthyl)-1-(cyclohexylméthyl)-3-phényl-1H-pyrazole-5-carboxylate d'éthyle

On ajoute 25 mg d'hydrure de sodium à 192 mg du produit obtenu au stade 2 ci-dessus, dans 2 ml de diméthylformamide puis agite 15 minutes à température ambiante. On ajoute 0,087 ml de 1-bromo (1-cyclohexyl) méthyle, agite 20 heures, verse dans l'eau, extrait à l'acétate d'éthyle, lave avec une solution aqueuse de chlorure de sodium, sèche et évapore le solvant sous pression réduite. On chromatographie le résidu sur silice (éluant : chlorure de méthylène-acétate d'éthyle : 95-5) et obtient le produit attendu soit 138 mg d'isomère A, F = 130°C et 39 mg d'isomère B (amorphe).

### STADE 4 : Acide 4-((6-chloro-1,3-benzodioxol-5-yl) méthyl)-1-(cyclohexylméthyl)-3-phényl-1H-pyrazole-5-carboxylique

On agite 16 heures à température ambiante 120 mg de l'isomère A obtenu au stade 3 ci-dessus, dans 5 ml d'éthanol en présence de 0,46 ml de soude 2N. On évapore les solvants, reprend dans 5 ml d'eau, filtre, neutralise par addition d'acide chlorhydrique 2N, filtre le précipité, le rince à l'eau et recueille 90 mg de produit attendu.
F = 234°C.

| Spectre IR (CHCl₃ cm⁻¹) | |
|---|---|
| C=O | 1686 |
| Hétérocycle + aromatique | 1600-1520-1504 |

### EXEMPLE 2 : Acide 1-butyl-5-((6-chloro-1,3-benzodioxol-5-yl) méthyl)-3-((5-carboxypentyl) thio)-1H-pyrazole-4-carboxylique

### STADE 1 : 6-chloro-1,3-benzodioxole-5-acétonitrile

On introduit 1,025 g de chlorure de 6-chloropipéronyl, 5 ml de diméthylformamide, 330 mg de cyanure de potassium, agite à température ambiante durant 6 heures, verse dans 50 ml d'eau, extrait par 3 x 30 ml d'acétate d'éthyle, lave par 30 ml de chlorure de sodium et sèche. On recristallise dans 5 ml d'éther isopropylique à reflux, essore et obtient 740 mg de produit attendu (cristaux blancs).

| ANALYSE : | |
|---|---|
| IR CHCl₃ cm⁻¹ | |
| CN | 2255 |
| Aromatique | 1625-1505-1481 |

### STADE 2 : 6-chloro-béta-oxo-1,3-benzodioxole-5-butanoate d'éthyle

On introduit 8,3 g de zinc électrolytique et 10 ml de tétrahydrofuranne. On active le zinc par addition de 0,2 ml de 1,2-dibromoéthane et 5 minutes de reflux. Après refroidissement, on ajoute 5 g du produit obtenu ainsi qu'il est indiqué au stade 1 ci-dessus, 40 ml de tétrahydrofuranne, amène à reflux et introduit en 1 heure, 11,4 ml de bromo acétate d'éthyle, agite encore 15 minutes à reflux, refroidit à 0+5°C et hydrolyse par 25 ml d'acide chlorhydrique. Après 30 minutes d'agitation, on verse dans 250 ml d'eau, extrait par 100 + 2 x 50 ml d'acétate d'éthyle, lave par 50 ml de chlorure de sodium et sèche. On recristallise dans l'éthanol 96 %, puis chromatographie sur silice avec pour éluant du chlorure de méthylène-acétate d'éthyle : 95-5. On obtient ainsi 885 mg de produit attendu.

| ANALYSE : | |
|---|---|
| IR CHCl₃ cm⁻¹ | |
| C + O | 1741-1720 |
| Aromatique | 1625-1507-1482 |

### STADE 3 : alpha-[bis(méthylthio)méthylène]-6-chloro-béta-oxo-1,3-benzodioxole-5-butanoate d'éthyle

### a) Préparation de fluorure de potassium sur alumine

On dissout 20 g de fluorure de potassium dans 200 ml d'eau, puis ajoute 30 g d'alumine active neutre. Après quelques minutes d'agitation, on sèche en reprenant plusieurs fois par de l'éthanol 100 % puis sèche. On obtient ainsi 50 g de fluorure de potassium sur alumine.

### b) Obtention du cétène thioacétal

On introduit 570 mg du produit obtenu au stade 2 ci-dessus, 20 ml d'acétonitrile, 1,6 g de produit préparé ci-dessus en a), 0,12 ml de disulfure de carbone, agite à température ambiante durant 1 heure puis introduit 0,25 ml d'iodure de méthyle et agite à température ambiante durant 16 heures. On filtre, rince 3 fois à l'acétonitrile et sèche. On reprend par 20 ml de chlorure de méthylène, filtre et sèche. On chromatographie sur silice dans du chlorure de méthylène et obtient ainsi 600 mg de produit attendu (solide jaune).
F = 70°C.

| ANALYSE : | |
|---|---|
| IR CHCl₃-Nujol cm⁻¹ | |
| C=O | 1704 |
| C=C + Aromatique | 1627-1526-1505-1482 |

### STADE 4 : 1-butyl-3-((6-chloro-1,3-benzodioxol-5-yl)méthyl)-5-(méthylthio)-1H-pyrazole-4-carboxylate d'éthyle (A) 1-butyl-2-((6-chloro-1,3-benzodioxol-5-yl)méthyl)-4-(méthylthio)-1H-pyrazole-3-carboxylate d'éthyle (B)

On introduit 150 mg du produit obtenu au stade 3 ci-dessus, 5 ml d'éthanol, 136 mg de butylhydrazine oxalate, amène à reflux et agite durant 3 jours et sèche. On chromatographie sur silice avec pour éluant du cyclohexane-acétate d'éthyle : 9-1. On obtient ainsi 21 mg du produit attendu A (résine) et 45 mg du produit attendu B.

| ANALYSE : Produit A | |
|---|---|
| IR CHCl₃ cm⁻¹ | |
| C=O | 1702 |
| Aromatique + Hétérocycle | 1625-1505-1483 |

### STADE 5 : 1-butyl 5-((6-chloro 1,3-benzodioxol-5-yl) méthyl) 3-(méthylsulfinyl)-1H-pyrazole-4-carboxylate d'éthyle.

On dissout 250 mg de l'ester éthylique obtenu à partir du produit obtenu ainsi qu'il est indiqué au stade 4 ci-dessus, dans 5 ml de dichlorométhane, refroidit à 0°C et ajoute 180 mg d'acide métachloroperbenzoïque en solution dans 8 ml de dichlorométhane et agite 45 minutes. On lave le milieu réactionnel avec une solution de bicarbonate de sodium, extrait au dichlorométhane, lave à l'eau la phase organique, la sèche et évapore le solvant sous pression réduite. On obtient le produit brut que l'on chromatographie sur silice (éluant : dichlorométhane-méthanol 97,5-2,5). On obtient 178 mg de produit attendu. F = 82°C.

### STADE 6 : 1-butyl-5-((6-chloro-1,3-benzodioxol-5-yl) méthyl) 3-mercapto-1H-pyrazole-4-carboxylate d'éthyle.

On dissout 169,3 mg de produit obtenu au stade 5 ci-dessus dans 20 ml dé dichlorométhane et ajoute 0,11 ml d'anhydride trifluoroacétique. On agite pendant 15 minutes, évapore le solvant sous pression réduite, reprend le résidu dans 2 ml de méthanol et 0,55 ml de triéthylamine et agite de nouveau 15 minutes à température ambiante, extrait au dichlorométhane, lave la phase organique avec une solution aqueuse saturée en chlorure d'ammonium, sèche et évapore le solvant sous pression réduite et récupère 125 mg de produit attendu.

### STADE 7 : 1-butyl 5-((6-chloro 1,3-benzodioxol-5-yl) méthyl) 3-((6-éthoxycarbonylpentyl)-thio)-1H-pyrazole-4-carboxylate d'éthyle.

On ajoute 14,4 mg d'hydrure de sodium dans une solution comprenant 125 mg de produit obtenu comme au stade 6 ci-dessus dans 2 ml de diméthylformamide. On agite 15 minutes et ajoute ensuite 0,056 ml d'éthyl-6-bromo hexanoate et agite 5 heures à température ambiante. On ajoute 4 ml d'eau, extrait à l'acétate d'éthyle, lave avec une solution aqueuse saturée en chlorure de sodium, sèche et évapore le solvant sous pression réduite. Après chromatographie sur silice (éluant : chlorure de méthylène-acétate d'éthyle : 95-5), on obtient 82,6 mg de produit attendu.

### STADE 8 : Acide 1-butyl 5-((6-chloro-1,3-benzodioxol-5-yl) méthyl)-3-((5-carboxypentyl) thio)-1H-pyrazole-4-carboxylique.

On mélange 72,4 mg de produit obtenu au stade 7 ci-dessus dans 0,26 ml de soude 2N et 2 ml d'éthanol. On agite 3 heures au reflux, évapore les solvants organiques sous pression réduite, ajoute 4 ml d'eau, acidifie la solution à l'aide de 0,26 ml d'acide chlorhydrique 2N, essore le précipité formé, le lave à l'eau, le sèche à 40°C sous pression réduite et récupère 49,2 mg de produit attendu. F = 140°C.

### EXEMPLE 3 : Acide 1-butyl-5-((6-chloro-1,3-benzodioxol-5-yl) méthyl)-3-(((4-méthoxyphényl) méthyl) thio)-1H-pyrazole-4-carboxylique

On procède comme à l'exemple 2 en utilisant au stade 7 de l'exemple 2 du 1-chloro-((4-méthoxyphényl) méthyl) au lieu d'éthyl-6-bromohexanoate et obtient ainsi le produit attendu. F = 180.9°C.

### EXEMPLE 4 : Acide 4-((6-chloro-1,3-benzodioxol-5-yl) méthyl) 1-(2-cyclohexyléthyl)-3-phényl)-1H-pyrazole 5-carboxylique

On procède comme à l'exemple 1 en utilisant au stade 3 de l'exemple 1 du 1-bromo-(2-cyclohexyl) éthyl) au lieu du 1-bromo (1-cyclohexyl) méthyle puis en procédant comme au stade 4 de l'exemple 1 et obtient ainsi le produit attendu. F ≃ 80°C.

### EXEMPLE 5 : Acide 1-((3-carboxycyclohexyl) méthyl)-4-((6-chloro-1,3-benzodioxol-5-yl) méthyl)-3-phényl-1H-pyrazole-5-carboxylique

On procède comme à l'exemple 1 en utilisant au stade 3 de l'exemple 1 du 1-[(3-tosylméthyl) cyclohexyl] carboxylate d'éthyle préparé comme indiqué ci-dessus au lieu du 1-bromo (1-cyclohexyl) méthyle puis en procédant comme au stade 4 de l'exemple 1 et obtient ainsi le produit attendu.
F - 130°C.

### Préparation du tosylate utilisé à l'exemle 5 :

### - formation du chlorure d'acide

On agite 3 heures au reflux 5 g d'acide 1,3-cyclohexane dicarboxylique dans 10 ml de chlorure de thionyle, distille l'excès de réactif, reprend par du toluène, évapore le solvant et récupère 6,04 g de produit attendu.

### - formation de l'ester

On refroidit à 0°C 1,69 ml d'éthanol et 50 ml de tétrahydrofuranne, ajoute en 30 minutes 18,12 ml de n-butyllithium en solution dans du tétrahydrofuranne (1,6 M/l) et agite 30 minutes à 0°/+5°C. On ajoute cette solution en 30 minutes à 6,04 g du chlorure d'acide obtenu précédemment dans 60 ml de tétrahydrofuranne à une température inférieure à 10°C.

### - réduction en alcool

En maintenant cette température, on ajoute en 30 minutes 58 ml de lithium tri(terbutoxyalumino hydrure), agite 1 heure, verse dans l'acide chlorhydrique N, extrait à l'acétate d'éthyle, lave à l'eau salée, sèche, évapore sous pression réduite, chromatographie le résidu (éluant : CH₂Cl₂/AcOEt 9-1) et obtient 1,37 g d'alcool.

### - formation du tosylate

On refroidit à 0°/+5°C 1,33 g de l'alcool précédent dans 5 ml de pyridine, ajoute en 20 minutes 1,63 g de chlorure de tosyle dans 5 ml de dichlorométhane et agite 20 heures à température ambiante. On verse le milieu réactionnel dans 100 ml d'acide chlorhydrique N, extrait à l'acétate d'éthyle, lave à l'eau, sèche, évapore le solvant sous pression réduite, chromatographie le résidu sur silice (éluant : CH₂Cl₂) et obtient 1,97 g de tosylate attendu.

### EXEMPLE 6 : Acide cis-1-[[2-[(carboxyméthoxy) cyclohexyl] méthyl]-4-((6-chloro-1,3-benzodioxol-5-yl) méthyl]-3-phényl-1H-pyrazole-5-carboxylique

On procède comme à l'exemple 1 en utilisant au stade 3 de l'exemple 1 du (1-tosyl)-1-[2-((éthoxycarbonylméthoxy) méthyl) cyclohexyl] méthyl au lieu du 1-bromo (1-cyclohexyl) méthyle puis en procédant comme au stade 4 de l'exemple 1 et obtient ainsi le produit attendu. F = 90°C.

### Préparation du tosylate utilisé à l'exemple 6 :

### - formation de l'ester

On ajoute en 1 heure à température ambiante 2,23 ml de diazoacétate d'éthyle en solution dans 16 ml de dichlorométhane à 3,06 g de cis 1,2-cyclohexane diméthanol dans 100 ml de dichlorométhane en présence de quelques gouttes de borotrifluorure d'éthyl éthérate. On maintient 16 heures à température ambiante sous agitation, lave le milieu réactionnel à l'eau, sèche, évapore le solvant sous pression réduite, chromatographie le résidu sur silice (éluant : AcOEt/cyclohexane 20-80) et obtient 1,98 g de produit attendu.

### - formation du tosylate

On refroidit à 0°/+5°C 1,01 g de produit précédent dans 5 ml de pyridine, ajoute en 30 minutes 1,00 g de chlorure de tosyle dans 5 ml de dichlorométhane et agite 16 heures à température ambiante. On verse le milieu réactionnel dans 100 ml d'acide chlorhydrique N, extrait au dichlorométhane, lave à l'eau, sèche, évapore le solvant sous pression réduite, chromatographie le résidu sur silice (éluant : cyclohexane/AcOEt 2-8) et obtient 1,38 g de tosylate attendu.

### EXEMPLE 7 : Acide 1-[[1-[(carboxyméthoxy) méthyl] cyclohexyl] méthyl]-4-((6-chloro-1,3-benzodioxol-5-yl) méthyl]-3-phényl-1H-pyrazole-5-carboxylique

On procède comme à l'exemple 1 en utilisant au stade 3 de l'exemple 1 du (1-bromo)-1-[1-((éthoxycarbonylméthoxy) méthyl) cyclohexyl] méthyl au lieu du 1-bromo (1-cyclohexyl) méthyle puis en procédant comme au stade 4 de l'exemple 1 et obtient ainsi le produit attendu. F = 188°C.

### Préparation du tosylate utilisé à l'exemple 7 :

On opère comme pour la préparation du tosylate utilisé à l'exemple 6 en utilisant au départ 1,09 ml de diazoacétate d'éthyle et 1,5 g de 1,1-(cyclohexane) diméthanol et obtient 840 mg d'ester intermédiaire. On fait réagir 820 mg de cet ester avec 814 mg de chlorure de tosyle comme indiqué ci-dessus à l'exemple 6 pour obtenir 1,23 g de tosylate attendu.

### EXEMPLE 8 : Acide 4-((6-chloro-1,3-benzodioxol-5-yl), méthyl) 1-cyclohexylméthyl)-3-(2-thiényl)-1H-pyrazole-5-carboxylique

### STADE 1 : (E) 3-((6-chloro-1,3-benzodioxol-5-yl)-2-propènoate d'éthyle

On ajoute goutte à goutte 60 ml de triéthylphosphonoacétate à 11,6 g d'hydrure de sodium dans 100 ml de diméthyléther d'éthylène glycol. On agite 50 minutes puis, en maintenant à la température ambiante, on ajoute 17,37 ml de 6-chloro pipéronal dans 370 ml de diméthyléther d'éthylène glycol, agite 2 heures et demie, filtre le précipité et évapore à sec le filtrat et cristallise le résidu dans l'éthanol. On obtient 46,33 g de produit attendu. F = 121°C.

### STADE 2 : 6-chloro-1,3-benzodioxol-5-propanoate d'éthyle

On refroidit à 0°C 43,93 g de l'ester obtenu au stade 1 ci-dessus dans 900 ml de tétrahydrofuranne et 300 ml d'éthanol puis ajoute 25,61 g de chlorure cuivreux puis en 30 minutes 6,52 g de borohydrure de sodium. On agite 2 heures à 0°C puis verse dans 1 litre de solution aqueuse glacée à 3% d'acide chlorhydrique. On agite 16 heures à température ambiante, filtre, lave à l'eau, extrait au chlorure de méthylène, sèche, évapore le solvant sous pression réduite, chromatographie le résidu sur silice (éluant : cyclohexane-acétate d'éthyle, 8-2) et obtient 25,21 g de produit attendu.

### STADE 3 : 2-((6-chloro-1,3-benzodioxol-5-yl) méthyl)-3-oxo 1,4-butanedioate de diéthyle

On chauffe 30 minutes à 80°C, 22,88 g du produit obtenu au stade 2 ci-dessus, dans 230 ml de toluène en présence de 18,96 g d'éthylate de sodium. On ajoute 18 ml de diéthyloxalate, agite 1 heure à 80°C, refroidit à 0°C puis verse dans 500 ml de solution aqueuse glacée saturée en chlorure d'ammonium additionné de 500 ml d'acétate d'éthyle. On laisse revenir à température ambiante, extrait la phase aqueuse à l'acétate d'éthyle, lave les phases organiques à l'eau salée, sèche, évapore le solvant sous pression réduite, chromatographie le résidu sur silice (éluant : cyclohexane-acétate d'éthyle : 8-2) et recueille 24,05 g de produit attendu.

### STADE 4 : 4-((6-chloro 1,3-benzodioxol-5-yl) méthyl) 1-(2-cyanoéthyl) 5-hydroxy 1H-pyrazole 3-carboxylate d'éthyle

On chauffe 7 heures au reflux 10,06 g de produit obtenu au stade 3 ci-dessus, dans 100 ml d'acide acétique glacial en présence de 2-cyano-éthylhydrazine. On laisse revenir à température ambiante et agite 16 heures. On filtre le précipité, le lave à l'éthanol, le sèche. Le filtrat est évaporé à sec, chromatographie le résidu sur silice (éluant : chlorure de méthylène-méthanol : 9-1) et reprend le résidu et le précipité séché dans 90 ml d'éthanol au reflux. On filtre, sèche sous pression réduite et récupère 5,49 g de produit attendu. F = 192°C.

### STADE 5 : 4-((6-chloro 1,3-benzodioxol-5-yl) méthyl) 1-(2-cyanoéthyl) 5-bromo 1H-pyrazole 3-carboxylate d'éthyle

On introduit 26,3 g de tribromophosphate, chauffe à 60°C, rajoute 5,32 g du produit obtenu au stade 4 ci-dessus et agite 20 minutes à 60°C. On dilue par 50 ml d'acétate d'éthyle, verse dans 500 ml de solution NH₄OH + glace, extrait par 3 x 200 ml d'acétate d'éthyle, lave par une solution saturée de chlorure de sodium et sèche. Après chromatographie sur silice dans cyclohexane-acétate d'éthyle : 5-5, on obtient 4,84 g de produit attendu (solide blanc).
F = 116°C.

| ANALYSES | |
|---|---|
| Spectre IR : dans CHCl₃ (cm⁻¹) | |
| Absence d'OH | |
| CN | 2258 |
| C=O | 1723 |
| Aromatique + hétérocycle | 1622 - 1600 - 1535 - 1505 - 1482 |

### STADE 6 : 4-((6-chloro-1,3-benzodioxol-5-yl) méthyl)-1-cyclohexylméthyl)-3-(2-bromo)-1H-pyrazole 5-carboxylate d'éthyle

On introduit 450 mg d'hydrure de sodium à 50 % dans l'huile, 3 ml de diméthylformamide, amène à 0°C puis introduit en 10 minutes, 4,125 g du produit obtenu au stade 5 ci-dessus et 8 ml de diméthylformamide. On agite à 0°C pendant 3/4 d'heure, puis rajoute 9 ml de diméthylformamide, agite encore 3/4 d'heure, puis rajoute progressivement 3,35 ml de bromométhylcyclohexane, laisse revenir à température ambiante, chauffe 6 h 30 à (55 ± 5)°C et agite 18 h encore à température ambiante. On verse dans 200 ml de solution saturée en chlorure d'ammonium, extrait par 3 x 200 ml d'acétate d'éthyle, lave par une solution saturée en chlorure de sodium et sèche. Après chromatographie sur silice dans le chlorure de méthylène puis dans cyclohexane-acétate d'éthyle : 9-1, on obtient 2,50 g de produit attendu (cristaux blancs). F = 116°C.

| ANALYSES | |
|---|---|
| Spectre IR : dans CHCl₃ (cm⁻¹) | |
| Absence de CN | |
| C=O | 1718 |
| Aromatique + hétérocycle. | 1625 - 1600 - 1527 - 1505 - 1480. |

### STADE 7 : 4-((6-chloro-1,3-benzodioxol-5-yl) méthyl)-1-cyclohexylméthyl)-3-(2-thiényl)-1H-pyrazole 5-carboxylate d'éthyle

On introduit 5 ml de diméthylformamide (anhydre), 96 mg d'acide 2-thiophèn-boronique, 17 mg de Pd(PΦ₃)₄ et 212,3 mg de K₃PO₄ puis 241,9 mg du produit obtenu au stade 6 ci-dessus et porte le milieu réactionnel à 100°C pendant 24 h.

On ajoute de l'acétate d'éthyle, lave la phase organique avec H₂O saturée de chlorure d'ammonium, à l'eau puis à l'eau saturée de chlorure de sodium, sèche, filtre et évapore à sec. Après chromatographie sur silice avec pour éluant chlorure de méthylène-cyclohexane : 1-1, on obtient 124,5 mg de produit attendu (solide-huileux).

| ANALYSES | |
|---|---|
| Spectre IR : dans CHCl₃ (cm⁻¹) | |
| >=O | 1714 |
| Aromatique + hétérocycle | 1555 - 1520 - 1504 - 1480 |

### STADE 8 : Acide 4-((6-chloro-1,3-benzodioxol-5-yl) méthyl)-1-cyclohexylméthyl)-3-(2-thiényl)-1H-pyrazole 5-carboxylique

On introduit 2,8 ml d'éthanol, 114,6 mg du produit obtenu au stade 7 ci-dessus, ajoute 284 µl de soude (2N) et porte le milieu au reflux pendant 2 heures. On évapore l'éthanol, ajoute 15 ml d'H₂O distillée, filtre, puis neutralise avec 284 µl d'acide chlorhydrique (2N). La suspension est laissée à température ambiante pendant 1 nuit, 10 minutes à 50°C, et 5 minutes sous ultrason. On filtre, lave à l'eau et sèche. On obtient ainsi 90,9 mg de produit attendu. F = 254°C.

| ANALYSES | | |
|---|---|---|
| Microanalyses : C₂₃H₂₃ClN₂O₄S : PM 458,97 | | |
| | Calculés | Trouvés |
| C % | 60,19 | 60,5 |
| H % | 5,05 | 5,2 |
| N % | 6,10 | 5,8 |
| S % | 6,99 | 6,7 |
| Cl % | 7,72 | 7,6 |
| Spectre IR : Nujol (cm⁻¹) | | |
| Absorption OH/NH | | |
| C=O | | 1686 |
| Aromatique + hétérocycle | | 1518 - 1500. |

### EXEMPLE 9 : Acide 4-((6-chloro-1,3-benzodioxol-5-yl) méthyl) 1-(cyclohexylméthyl)-3-(2-pyridinyl)-1H-pyrazole-5-carboxylique

### STADE 1 : 4-((6-chloro-1,3-benzodioxol-5-yl) méthyl)-1-(cyclohexylméthyl)-3-(2-pyridinyl)-1H-pyrazole 5-carboxylate d'éthyle

On introduit 10 ml de toluène (anhydre), 368,4 mg de 2-(tributyl-stannyl)-pyridine et 29 mg de Pd(PΦ₃)₄ puis rapidement 241,9 mg du produit obtenu au stade 6 de l'exemple 8, et porte le milieu au reflux pendant 1 nuit.

On ajoute de l'acétate d'éthyle, lave la phase organique avec H₂O saturée de chlorure d'ammonium, à l'eau puis à l'eau saturée de chlorure de sodium, sèche, filtre et évapore à sec. Après chromatographie sur silice avec pour éluant acétate d'éthyle-chlorure de méthylène : 10-90, on obtient 174,7 mg de produit attendu.

| ANALYSES | |
|---|---|
| Spectre IR : dans CHCl₃ (cm⁻¹) | |
| >=O | 1710 |
| Aromatique + hétérocycle | 1592 - 1568 - 1522 - 1504 - 1480. |

### STADE 2 : Acide 4-((6-chloro-1,3-benzodioxol-5-yl) méthyl)-1-(cyclohexylméthyl)-3-(2-pyridinyl)-1H-pyrazole-5-carboxylique

On introduit 3,4 ml d'éthanol, 164 mg du produit obtenu au stade 1 ci-dessus, 340 µl de soude (2N) et porte le milieu à 80°C pendant 2 heures. On évapore pour éliminer l'éthanol, ajoute 12 ml d'eau distillée, rajoute 340 µl d'acide chlorhydrique (2N), laisse la suspension sous agitation pendant 24 heures, puis à 50°C pendant 1 h et 30 mn sous ultrason. On filtre, lave à l'eau et sèche. On obtient ainsi 140,8 mg de produit attendu (solide blanc). F = 238°C.

| ANALYSES | | |
|---|---|---|
| Microanalyses : C₂₃H₂₄ClN₃O₄ : PM 453,93 | | |
| | Calculés | Trouvés |
| C % | 63,50 | 63,2 |
| H % | 5,33 | 5,2 |
| Cl % | 7,81 | 7,8 |
| N % | 9,26 | 9,1 |
| Spectre IR : Nujol (cm⁻¹) | | |
| Absorption OH/NH | | |
| C=O | | 1688 |
| Aromatique + hétérocycle | | 1591 - 1568 - 1526 - 1500. |

### EXEMPLE 10 : Acide 4-((6-chloro-1,3-benzodioxol-5-yl) méthyl) 1-(cyclohexylméthyl)-3-(3-thiényl)-1H-pyrazole-5-carboxylique

### STADE 1 : 4-((6-chloro-1,3-benzodioxol-5-yl) méthyl) 1-(cyclohexylméthyl) 3-(3-thiényl)-1H-pyrazole 5-carboxylate d'éthyle

On opère comme au stade 7 de l'exemple 8 à partir de 5,0 ml de diméthylformamide (anhydre), 96 mg d'acide 3-thiophèn-boronique, 17 mg de Pd(PΦ₃)₄ et 212,3 mg de K₃PO₄, 241,9 mg du produit obtenu au stade 6 de l'exemple 8 et porte le milieu réactionnel à 100°C pendant 16 h. Après chromatographie sur silice, avec pour éluant chlorure de méthylène-cyclohexane : 1-1, on obtient 197,5 mg de produit attendu.

| ANALYSES | |
|---|---|
| Spectre IR : dans CHCl₃ (cm⁻¹) | |
| >=O | 1710 |
| Aromatique + hétérocycle | 1600 - 1560 - 1524 - 1505 - 1480. |

### STADE 2 : Acide 4-((6-chloro-1,3-benzodioxol-5-yl) méthyl)-1-(cyclohexylméthyl)-3-(3-thiényl)-1H-pyrazole 5-carboxylique

On procède comme au stade 8 de l'exempl 8 à partir de 3,94 ml d'éthanol, 186,9 mg du produit obtenu au stade 1 ci-dessus, 394 µl de soude (2N) et porte ensuite le milieu au reflux pendant 2 h 30. On obtient ainsi 165,3 mg de produit attendu (solide blanc). F = 250°C.

| ANALYSES | | |
|---|---|---|
| Microanalyses : C₂₃H₂₃ClN₂O₄S : PM 458,97 | | |
| | Calculés | Trouvés |
| C % | 60,19 | 60,3 |
| H % | 5,05 | 5,0 |
| N % | 6,10 | 5,9 |
| S % | 6,99 | 6,9 |
| Cl % | 7,72 | 7,7 |
| Spectre IR : Nujol (cm⁻¹) | | |
| Absorption OH/NH | | |
| C=O | | 1683 |
| Aromatique + hétérocycle | | 1520 - 1500. |

### EXEMPLE 11: Acide 1-butyl 3-((4-(carboxyméthyl) cyclohexyl) thio) 5-((6-chlore 1,3-benzodioxol-5-yl) méthyl) 1H-pyrazole 4-carboxylique.

On opère comme à l'exemple 2 en utilisant au stade 7 le pyrazole mercaptan brut obtenu comme au stade 6 et du 4-bromocyclohexyl acétate de terbutyle et en maintenant l'agitation pendant 20 heures. On obtient le produit attendu.
F = 100°C.

### EXEMPLE 12 : Acide 3-((5-carboxypentyl) thio) 5-((6-chloro 1,3-benzodioxol-5-yl) méthyl) 1-propyl 1H-pyrazole 4-carboxylique.

### Stade 1 : 2-((6-chloro 1,3-benzodioxol-5-yl) méthyl) 4-(méthylthio) 1H-pyrazole 3-carboxylate d'éthyle.

On mélange 500 mg du produit obtenu au stade 3 de l'exemple 2, 5 ml d'éthanol et 0,12 ml d'hydrate d'hydrazine, agite 1 heure à température ambiante, élimine le solvant et les vapeurs de méthylmercaptan et recueille 357 mg de produit brut après chromatographie sur silice (éluant : CH₂Cl₂-AcOEt 8-2). F = 63°C.

### Stade 2 : 1-propyl 3-((6-chloro 1,3-benzodioxol-5-yl) méthyl) 5-(méthylthio) 1H-pyrazole 4-carboxylate d'éthyle (A) 1-propyl 2-((6-chloro 1,3-benzodioxol-5-yl) méthyl) 4-méthylthio) 1H-pyrazole 3-carboxylate d'éthyle (B).

On mélange 12,85 g du produit obtenu comme au stade 1 ci-dessus, 200 ml de diméthylformamide et 1,74 g d'hydrure de sodium. On agite 15 minutes à température ambiante, ajoute 8,15 ml de bromopropane, agite 16 heures, verse dans l'eau, extrait à l'acétate d'éthyle, élimine le solvant sous pression réduite, chromatographie le résidu sur silice (éluant : cyclohexane-éther 8-2) et obtient 2,03 g de produit attendu, isomère A F = 50°C et 8,66 g de produit attendu, isomère B F = 94°C.

### Stade 3 : 1-propyl 5-((6-chloro 1,3-benzodioxol-5-yl) méthyl) 3-(méthylsulfinyl) 1H-pyrazole 4-carboxylate d'éthyle.

On opère comme au stade 5 de l'exemple 2 en utilisant au départ 8,66 g de produit obtenu au stade 2 ci-dessus et 6,46 g d'acide métachloroperbenzoïque. On obtient 7,39 g de produit attendu utilisé tel quel pour le stade suivant.

### Stade 4 : 1-propyl 5-((6-chloro 1,3-benzodioxol-5-yl) méthyl) 3-mercapto 1H-pyrazole 4-carboxylate d'éthyle.

On opère comme au stade 6 de l'exemple 2 en utilisant au départ 4,25 g de produit obtenu au stade 3 ci-dessus. On obtient 4,2 g de produit attendu.

### Stade 5 : 1-propyl 5-((6-chloro 1,3-benzodioxol-5-yl) méthyl) 3-((6-éthoxycarbonylpentyl) thio) 1H-pyrazole 4-carboxylate d'éthyle.

On opère comme au stade 7 de l'exemple 2 en utilisant au départ 685 mg du produit obtenu au stade 4 ci-dessus et 0,96 ml de 6-bromo hexanoate d'éthyle et en maintenant l'agitation pendant 20 heures. On obtient 250 mg de produit attendu.

### Stade 6 : Acide 3-((5-carboxypentyl) thio) 5-((6-chloro 1,3-benzodioxol-5-yl) méthyl) 1-propyl 1H-pyrazole 4-carboxylique.

On opère comme au stade 8 de l'exemple 2 en utilisant au départ 250 mg de diester préparé au stade 5 ci-dessus. On obtient 137 mg de produit attendu. F = 88°C.

### EXEMPLE 13 : Acide 3-((4-(carboxyméthyl) cyclohexyl) thio) 5-((6-chloro 1,3-benzodioxol-5-yl) méthyl) 1-propyl 1H-pyrazole 4-carboxylique.

On opère comme à l'exemple 12 en faisant réagir, au stade 5, sur le 1-propyl 5-((6-chloro 1,3-benzodioxol-5-yl) méthyl) 3-mercapto 1H-pyrazole 4-carboxylate d'éthyle, du 4-bromocyclohexyl éthylate de terbutyle. On obtient le produit attendu. F = 178'C.

### EXEMPLE 14 : 3-((3-carboxypropyl) thio) 5-((6-chloro 1,3-benzodioxol-5-yl) méthyl) 1-propyl 1H-pyrazole 4-carboxylate d'éthyle.

On opère comme à l'exemple 12 en faisant réagir, au stade 5, sur le 1-propyl 5-((6-chloro 1,3-benzodioxol-5-yl) méthyl) 3-mercapto 1H-pyrazole 4-carboxylate d'éthyle, du 4-bromobutyrate d'éthyle. On obtient le produit attendu.
F = 110°C.

### EXEMPLE 15 : Acide 3-((4-carboxybutyl) thio) 5-((6-chloro 1,3-benzodioxol-5-yl) méthyl) 1-propyl 1H-pyrazole 4-carboxylique.

On opère comme à l'exemple 12 en faisant réagir, au stade 5, sur le 1-propyl 5-((6-chloro 1,3-benzodioxol-5-yl) méthyl) 3-mercapto 1H-pyrazole 4-carboxylate d'éthyle, du 5-bromo valérate d'éthyle. On obtient le produit attendu.
F = 185°C.

### EXEMPLE 16 : Acide 3-((6-carboxyhexyl) thio) 5-((6-chloro 1,3-benzodioxol-5-yl) méthyl) 1-propyl 1H-pyrazole 4-carboxylique.

On opère comme à l'exemple 12 en faisant réagir, au stade 5, sur le 1-propyl 5-((6-chloro 1,3-benzodioxol-5-yl) méthyl) 3-mercapto 1H-pyrazole 4-carboxylate d'éthyle, du 7-bromo heptanoate d'éthyle. On obtient le produit attendu.
F = 180°C.

### EXEMPLE 17 : Acide 1-((4-(carboxyméthyl) phényl) méthyl) 4-((6-chloro 1,3-benzodioxol-5-yl) méthyl) 3-phényl 1H-pyrazole 5-carboxylique.

### Stade A : 1-(((4-éthoxycarbonylméthyl) phényl) méthyl) 4-((6-chloro 1,3-benzodioxol-5-yl) méthyl) 3-phényl 1H-pyrazole 5-carboxylate d'éthyle et isomère 5-phényl correspondant.

### 1) Préparation de l'ester halogéné.

On ajoute lentement 2,44 ml de chlorure de triméthylsilyle à 2 g d'acide 4-bromo méthyl phényl acétique dans 40 ml d'éthanol et agite 2 heures à température ambiante. On évapore le solvant, reprend le résidu dans du dichlorométhane, lave à l'eau, sèche et évapore à sec sous pression réduite. On obtient 1,98 g de produit attendu. F < 45°C.

### 2) Couplage.

On ajoute 40 mg d'hydrure de sodium à 50% à 300 mg d'ester obtenu comme au stade 2 de l'exemple 1 dans 3 cm³ de diméthylformamide. On agite 15 minutes puis ajoute 218 mg de l'ester halogéné obtenu précédemment. Après 3 heures d'agitation à température ambiante, on verse dans l'eau, extrait à l'acétate d'éthyle et évapore le solvant sous pression réduite. On chromatographie le résidu sur silice (éluant : CH₂Cl₂-AcOEt 98-2) et obtient 206 mg de produit attendu sous forme d'isomère 3-phényl F # 90°C) et 92 mg sous forme d'isomère 5-phényl.

### Stade B : Acide 1-((4-(carboxyméthyl) phényl) méthyl) 4-((6-chloro 1,3-benzodioxol-5-yl) méthyl) 3-phényl 1H-pyrazole 5-carboxylique.

En opérant comme à l'exemple 1 stade 4 à partir de 186 mg de l'isomère 3-phényl obtenu au stade A ci-dessus, on a obtenu 154 mg de produit attendu. F = 250°C.

### EXEMPLE 18 : Acide 1-((4-carboxycyclohexyl) méthyl) 4-((6-chloro 1,3-benzodioxol-5-yl) méthyl) 3-phényl 1H-pyrazole 5-carboxylique.

On opère comme à l'exemple 17, en utilisant à la place de l'ester halogéné du 1-((4-tosyloxyméthyl) cyclohexyl) carboxylate d'éthyle et obtient le produit attendu.

### Préparation du tosylate.

On agite 5 heures à température ambiante 1 g de 1,4-dihydroxyméthyl cyclohexane dans 10 ml de diméthylformamide en présence de 0,94 g d'imidazole et 1,8 ml de chlorure de diphényl triméthyl silane puis verse dans une solution aqueuse saturée en chlorure d'ammonium, extrait à l'acétate d'éthyle, lave à l'eau salée, sèche, évapore le solvant sous pression réduite, chromatographie le résidu sur silice (éluant : CH₂Cl₂-AcOEt 95-5). Ob obtient 1,31 g de dérivé silylé. On agite à température ambiante pendant 40 heures 13,06 g de dérivé silylé préparé comme indiqué ci-dessus dans 260 ml de diméthylformamide avec 45 g de dichromate de pyridinium, concentre partiellement sous pression réduite, verse dans 400 ml d'acide chlorhydrique (N) glacé, extrait à l'acétate d'éthyle, lave avec une solution de bicarbonate de sodium, sèche, évapore le solvant sous pression réduite, obtient 13,1 g d'acide brut. On agite 70 heures à température ambiante 12,53 g de l'acide obtenu ci-dessus dans 125 ml d'éthanol 100° en présence de 10 ml de chlorotriméthylsilane puis évapore les solvants sous pression réduite. Après chromatographie sur silice (éluant CH₂Cl₂-AcOEt 95-5 à 80-20), on obtient 3,40 g d'ester débloqué.

### Formation du tosylate.

On refroidit à 0°/+5°C 3,40 g du dérivé précédent dans 10 ml de pyridine, ajoute en 50 minutes 5,22 g de chlorure de tosyle dans 25 ml de dichlorométhane et agite 20 heures à température ambiante. On verse le milieu réactionnel dans 200 ml d'acide chlorhydrique N, extrait au dichlorométhane, lave à l'eau, sèche, évapore le solvant sous pression réduite, chromatographie le résidu sur silice (éluant : cyclohexane/AcOEt 2-8) et obtient 5,50 g de tosylate attendu.

### EXEMPLE 19 : Acide (cis/trans) 1-((4-(carboxyméthyl) cylohexyl) méthyl) 4-((6-chloro 1,3-benzodioxol-5-yl) méthyl) 3-phényl 1H-pyrazole 5-carboxylique.

En opérant comme à l'exemple 17 en utilisant le tosylate préparé ci-dessous à la place de l'halogénure, on obtient le produit attendu.

### Préparation du 1-cyanométhyl 4-tosyloxyméthyl cyclohexane utilisé au départ de l'exemple 19.

### 1) Formation du ditosylate.

On refroidit à 0°/+5°C 10 g de 1,4-cylcohexane diméthanol dans 50 ml de pyridine, ajoute en 1 heure 32 g de chlorure de tosyle dans 150 ml de dichlorométhane puis agite à température ambiante pendant 48 heures. On verse le milieu réactionnel sur 500 ml d'acide chlorhydrique 2N, extrait au dichlorométhane, lave à l'eau, sèche et évapore le solvant sous pression réduite. Après recristallisation du résidu dans un mélange dichlorométhane/acétate d'éthyle, on obtient 16,28 g de produit attendu. F = 165°C.

### 2) Préparation du produit cyané.

On agite pendant 3 jours à température ambiante, 4,52 g du tosylate préparé ci-dessus dans 90 ml de diméthylformamide en présence de 490 mg de cyanure de sodium. On verse le milieu réactionnel dans l'eau additionné de bicarbonate de sodium, extrait à l'acétate d'éthyle, lave à l'eau, sèche et évapore le solvant sous pression réduite. On obtient, après chromatographie sur silice (éluant : cyclohexane-CH₂Cl₂-AcOEt 5-4-1), 1,4 g de réactif attendu. F # 75'C.

### EXEMPLE 20 : Acide 1-((bicyclo(2.2.2)oct-2-yl) méthyl 4-((6-chloro 1,3-benzodioxol-5-yl) méthyl) 3-phényl 1H-pyrazole 5-carboxylique.

En opérant comme à l'exemple 17 en utilisant le 1-tosyloxyméthyl bicyclo(2.2.2)octane à la place de l'ester halogéné, on obtient le produit attendu. F = 120°C.

### EXEMPLE 21 : Acide 1-((4-(carboxycyclohexyl) méthyl) 4-((6-chloro 1,3-benzodioxol-5-yl) méthyl) 3-(2-thiényl) 1H-pyrazole 5-carboxylique.

### Stade A : α,γ-dioxo 2-thiophènebutanoate d'éthyle.

On refroidit à 0°C 20 g d'éthylate de sodium dans 600 ml d'éthanol puis ajoute lentement 15 ml de 2-acétyl thiophène, agite 2 heures à 0°C, ajoute en 25 minutes 28 ml de diéthyl oxalate, laisse revenir à température ambiante, agite 20 heures, refroidit de nouveau à 0°C puis hydrolyse par addition de 500 ml d'acide chlorhydrique (N). Après retour à température ambiante, on évapore l'éthanol sous pression réduite, extrait à l'acétate d'éthyle, lave à l'eau salée, sèche, évapore le solvant sous pression réduite, obtient 31,3 g de produit attendu après chromatographie sur silice (éluant: CH₂Cl₂-AcOEt 100% à 50%). F < 37°C.

### Stade B : β-((6-chloro 1,3-benzodioxol-5-yl) méthyl)-α,γ-dioxo-2-thiophènebutanoate d'éthyle.

On ajoute 10 g d'éthylate de sodium à 31,11 g de produit obtenu au stade A dans 155 ml de diméthylformamide, agite 15 minutes, puis ajoute 28,2 g de chlorure de 6-chloropipéronyle et 20,6 g d'iodure de sodium. Après 17 heures d'agitation à température ambiante, on verse le milieu réactionnel dans l'eau, extrait à l'acétate d'éthyle, élimine le solvant sous pression réduite.

### Stade C : 4-((6-chloro 1,3-benzodioxol-5-yl) méthyl) 3-(2-thiényl) 1H-pyrazole 5-carboxylate d'éthyle.

On chauffe à 80°C 53,23 g de produit brut obtenu ci-dessus dans 100 ml d'éthanol et ajoute 7 ml d'hydrate d'hydrazine et agite pendant 3 heures au reflux, laisse revenir à température ambiante, filtre le précipité, le lave à l'éthanol, le sèche sous pression réduite, le reprend dans l'éthanol au reflux, concentre sous pression réduite et recueille 7,97 g de produit attendu cristallisé.

### Stade D : 1-(((4-(éthoxycarbonyl) cylclohexyl) méthyl) 4-((6-chloro 1,3-benzodioxol-5-yl) méthyl) 3-(2-thiényl 1H-pyrazole 5-carboxylate d'éthyle.

On agite pendant 15 minutes 700 mg de produit obtenu au stade C ci-dessus dans 4,2 ml de diméthylformamide en présence de 112 mg d'hydrure de sodium à 50%. On ajoute à température ambiante 730 mg de 1-((4-tosyloxyméthyl) cyclohexyl) carboxylate d'éthyle préparé comme à l'exemple 18 dans 2 ml de diméthylformamide, agite 66 heures, ajoute de nouveau 305 mg de ce tosylate dans 1 ml de diméthylformamide, chauffe 3 heures à 80°C, laisse revenir à température ambiante, verse dans une solution aqueuse saturée en chlorure d'ammonium, extrait à l'acétate d'éthyle, sèche, évapore les solvants sous pression réduite, chromatographie le résidu sur silice (éluant : cyclohexane-acétate d'éthyle 9-1 et 8-2 puis CH₂Cl₂-AcOEt 99-1) et obtient après cristallisation dans l'êther 467 mg de produit attendu.

### Stade E : Acide 1-((4-(carboxycyclohexyl) méthyl) 4-((6-chloro 1,3-benzodioxol-5-yl) méthyl) 3-(2-thiényl) 1H-pyrazole 5-carboxylique.

On opère comme au stade 8 de l'exemple 2 en utilisant 245 mg de diester préparé ci-dessus et 0,88 ml de soude 2N. On obtient 124 mg de produit attendu. F = 285°C (décomp).

### EXEMPLE 22 : Acide cis/trans 1-((4-(carboxyméthyl) cyclohexyl) méthyl) 4-((6-chloro 1,3-benzodioxol-5-yl) méthyl) 3-(2-thiényl) 1H-pyrazole 5-carboxylique.

On opère comme à l'exemple 21 stades D et E en utilisant comme réactif tosylé le 1-cyanométhyl 4-tosyloxyméthyl cyclohexane préparé comme à l'exemple 19, et en effectuant la réaction à température ambiante pendant 16 heures. On obtient le produit attendu. F = 240°C.

### EXEMPLE 23 : Acide 1-((2-(carboxyméthoxy) phényl) méthyl) 4-((6-chloro 1,3-benzodioxol-5-yl) méthyl) 3-(2-thiényl) 1H-pyrazole 5-carboxylique.

On opère comme à l'exemple 21 stades D et E en utilisant à la place du réactif tosylé du chlorure de 2-((éthoxycarbonylméthoxy) benzyle). On obtient le produit attendu.
F > 260°C.

### EXEMPLE 24 : Acide 1-((3-carboxycyclohexyl) méthyl) 4-((6-chloro 1,3-benzodioxol-5-yl) méthyl) 3-(2-thiényl) 1H-pyrazole 5-carboxylique.

On opère comme à l'exemple 21 stades D et E en utilisant comme réactif tosylé le 1-((3-tosyloxyméthyl) cyclohexyl) carboxylate d'éthyle préparé comme indiqué à l'exemple 5 et en effectuant la réaction à température ambiante. On obtient le produit attendu. F > 260°C.

### EXEMPLE 25 : Acide 1-(2-carboxyméthoxy) 4-méthoxyphényl) 4-((6-chloro 1,3-benzodioxol-5-yl) méthyl) 3-(2-thiényl 1H-pyrazole 5-carboxylique.

On opère comme à l'exemple 21 stades D et E en utilisant à la place du réactif tosylé du chlorure de 4-méthoxy 2-((éthoxycarbonylméthoxy) benzyle). On obtient le produit attendu. F = 260°C.

### EXEMPLE 26 : Acide 4-((6-chloro 1,3-benzodioxol-5-yl) méthyl) 1-(cyclohexylméthyl) 3-(3-pyridinyl) 1H-pyrazole 5-carboxylique.

### Stade A : 4-((6-chloro 1,3-benzodioxol-5-yl) méthyl) 1-(cyclohexylméthyl) 3-(3-pyridinyl) 1H-pyrazole 5-carboxylate d'éthyle.

On ajoute sous atmosphère inerte 254 mg de dérivé bromé préparé comme indiqué au stade 6 de l'exemple 8, dans 10 ml de toluène à un mélange comprenant 386 mg de 3-(tributylstannyl) pyridine et 30 mg de tétrakistriphénylphosphine palladium. On chauffe 18 heures au reflux, ajoute de nouveau 30 mg de réactif palladié, maintient 4 heurs 30 au reflux puis verse sur 50 ml d'eau. On extrait au dichlorométhane, lave à l'eau, sèche, évapore le solvant sous pression réduite, chromatographie le résidu sur silice (éluant : cyclohexane-AcOEt 7-3) et obtient, après recristallisation dans un mélange éther-éther isopropylique, 144 mg de produit attendu. F = 135°C.

### Stade B : Acide 4-((6-chloro 1,3-benzodioxol-5-yl) méthyl) 1-(cyclohexylméthyl) 3-(3-pyridinyl) 1H-pyrazole 5-carboxylique.

On opère comme au stade 4 de l'exemple 1 en utilisant 130 mg d'ester obtenu au stade A et 0,27 ml de soude 2N. On recueille 98 mg de produit attendu. F = 144°C.

### EXEMPLE 27 : Acide 4-((6-chloro 1,3-benzodioxol-5-yl) méthyl) 1-(cyclohexylméthyl) 3-(2-furanyl) 1H-pyrazole 5-carboxylique.

On opère comme à l'exemple 26 stades A et B en utilisant au stade A 700 mg de dérivé bromé et 0,70 ml de 2-(tributylstannyl) furanne. On obtient 436 mg d'ester. On fait réagir 404 mg de cet ester et 0,86 ml de soude 2N et obtient 339 mg de produit attendu. F = 248°C.

### EXEMPLE 28 : Acide 1-((4-carboxycyclohexyl) méthyl) 4-((6-chloro 1,3-benzodioxol-5-yl) méthyl) 3-(2-pyridinyl) 1H-pyrazole 5-carboxylique.

### Stade A: α,γ-dioxo 2-pyridinebutanoate d'éthyle.

On refroidit à 0°C 3 g d'éthylate de sodium dans 120 ml d'éthanol, ajoute 6 ml de 2-acétyl pyridine, agite 5 minutes à 0°C puis ajoute en 5 minutes 12 ml de diéthyloxalate. On maintient sous agitation à 0°C pendant 1 heure, laisse revenir à température ambiante pendant 2 heures et demie, évapore le solvant sous pression réduite, reprend le résidu dans l'éther, filtre et sèche sous pression réduite à 70°C. On obtient 8,84 g de produit attendu. F = 155°C.

### Stade B : β-((6-chloro 1,3-benzodioxol-5-yl) α,γ-dioxo 2-pyridinebutanoate d'éthyle.

On mélange 7,71 g du produit obtenu au stade précédent dans 20 ml de diméthylformamide puis ajoute 6,45 g de chlorure de 6-pipéronyle et 7 g d'iodure de sodium dans 40 ml de diméthylformamide. On agite pendant 4 heures et demie, verse le milieu réactionnel dans l'eau, extrait au dichlorométhane, lave à l'eau, sèche, et évapore le solvant sous pression réduite et obtient le produit attendu utilisé tel quel pour le stade suivant.

### Stade C : 4-((6-chloro 1,3-benzodioxol-5-yl) méthyl) 3-(2-pyridinyl) 1H-pyrazole 5-carboxylate d'éthyle.

On opère comme à l'exemple 21 stade C en utilisant 12,58 g du produit obtenu au stade B et 1,2 ml d'hydrate d'hydrazine et obtient 4,98 g de produit attendu.

### Stade D : 1-(((4-éthoxycarbonyl) cyclohexyl) méthyl) 4-((6-chloro 1,3-benzodioxol-5-yl) méthyl) 3-(2-pyridinyl) 1H-pyrazole 5-carboxylate d'éthyle.

On opère comme à l'exemple 21 stade D en utilisant 638 mg du produit obtenu au stade C et 700 mg de 1-((4-tosyloxyméthyl) cyclohexyl) carboxylate d'éthyle préparé comme à l'exemple 18. On obtient 456 mg de produit attendu.

### Stade E : Acide 1-((4-carboxycyclohexyl) méthyl) 4-((6-chloro 1,3-benzodioxol-5-yl) méthyl) 3-(2-pyridinyl) 1H-pyrazole 5-carboxylique.

On opère comme au stade 8 de l'exemple 2 en utilisant 423 mg du diester préparé ci-dessus et 1,53 ml de soude 2N. On obtient 262 mg de produit attendu. F = 261°C.

### EXEMPLE 29 : Composition pharmaceutique.

On a préparé des comprimés répondant à la formule suivante :

| | |
|---|---|
| Produit de l'exemple 8 | 50 mg |
| Excipient pour un comprimé terminé à (détail de l'excipient : lactose, talc, amidon, stéarate de magnésium). | 200 mg |

### RESULTATS PHARMACOLOGIQUES

### 1) ETUDE DE L'ACTIVITE SUR RECEPTEUR A DE L'ENDOTHELINE

On effectue une préparation membranaire à partir de coeur (ventricules) de rat. Le tissu est broyé au POLYTRON dans un tampon Tris 50 mM pH = 7,4.

Après 30 minutes à 25°C (B.M.) l'homogénat est centrifugé à 30000 g pendant 15 minutes (2 centrifugations avec reprise intermédiaire dans le tampon Tris pH. 7,4).

Les culots sont remis en suspension dans un tampon d'incubation (Tris 25mM, pepstatine A 5 microg/ml, aprotinine 3 microg/ml, PMSF 0,1 mM, EDTA 3mM, EGTA 1mM pH 7,4).

On répartit des aliquotes de 2 ml dans des tubes à hémolyse et ajoute de la ¹²⁵I endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10-6 M (en triple). On incube à 25°C pendant 60 minutes, remet au bain-marié à 0°C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits d'exemples sont données dans le tableau I ci-après, en nanomoles.

### Résultats :

**TABLEAU I**

| Produit des exemples | Récepteur A de l'endothéline CI₅₀ en nanomoles |
|---|---|
| 1 | 3,6 |
| 5 | 2,5 |
| 8 | 1,1 |
| 9 | 4,7 |
| 10 | 1,1 |

### 2) ETUDE DE L'ACTIVITE SUR RECEPTEUR B DE L'ENDOTHELINE

On effectue une préparation membranaire à partir de cortex postérieur plus cervelet de rat. Le tissu est broyé au POLYTRON dans un tampon Tris 50 mM pH = 7,4.
Après 30 minutes à 25°C (B.M.) l'homogénat est centrifugé à 30000 g pendant 15 minutes (2 centrifugations avec reprise intermediaire dans le tampon Tris pH. 7,4).
Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).
On répartit des aliquotes de 2 ml dans des tubes à hémolyse et ajoute de la ¹²⁵I endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.
La liaison non spécifique est déterminée par addition d'endothéline à 10⁻⁶ M (en triple). On incube à 25°C pendant 60 minutes, remet au bain-marié à 0°C, pendant 5 minutes, filtre sous pression réduite, rince au tampon Tris 7,4 et compte la radioactivité en présence du scintillant Triton.
Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits d'exemples sont données dans le tableau II ci-après, en nanomoles.

### Résultats :

**TABLEAU II**

| Produit de l'exemple | Récepteur B de l'endothéline CI₅₀ en nanomoles |
|---|---|
| 1 | 2,6 |
| 5 | 14,8 |
| 8 | 1,7 |
| 9 | 3,4 |
| 10 | 3,2 |

## Revendications

1. Produits de formule (I) : 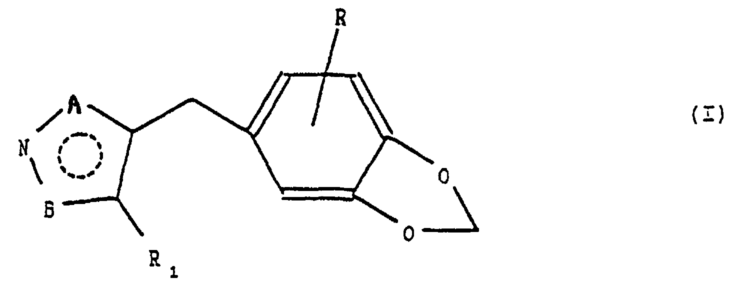 dans laquelle
A représente soit un atome d'azote substitué par un radical alkyle linéaire ou ramifié, renfermant au plus 4 atomes de carbone,
soit un radical méthine substitué par un radical phényle, thiényle ou pyridyle,
B représente soit un atome d'azote substitué par un radical alkyle renfermant au plus 4 atomes de carbone lui-même substitué par un radical cyclohexyle, lui-même éventuellement substitué par un radical carboxy ou par un radical carboxyméthoxyméthyle,
soit un radical méthine substitué par un radical alkylthio, renfermant au plus 6 atomes de carbone ou par un radical cyclohexylthio, ces radicaux étant éventuellement substitués par un radical carboxy ou par un radical phényle lui-même substitué par un radical alcoxy renfermant au plus 4 atomes de carbone, étant entendu que l'un de A et B représente un atome d'azote et l'autre de A et B représente un radical méthine, ces radicaux étant substitués comme indiqué ci-dessus,
R₁ représente un radical carboxy libre ou estérifié par un radical alkyle linéaire ou ramifié, renfermant au plus 4 atomes de carbone,
R représente un atome d'halogène,
ainsi que les produits suivants :
- acide 1-butyl 3-((4-(carboxyméthyl) cyclohexyl) thio) 5-((6(chloro 1,3-benzodioxol-5-yl) méthyl) 1H-pyrazole 4-carboxylique,
- acide 3-((4-(carboxyméthyl) cyclohexyl) thio) 5-((6-chloro 1,3-benzodioxol-5-yl) méthyl) 1-propyl 1H-pyrazole 4-carboxylique,
- acide 1-((bicyclo(2.2.2)oct-2-yl) méthyl) 4-((6-chloro 1,3-benzodioxol-5-yl) méthyl) 3-phényl 1H-pyrazole 5-carboxylique,
- acide cis/trans 1-((4-(carboxyméthyl)cyclohexyl)methyl) 4-((6-chloro 1,3-benzodioxol-5-yl) methyl) 3-(2-thiényl) 1H-pyrazole 5-carboxylique,
- acide 1-((2-(carboxyméthoxy) phényl) méthyl) 4-((6-chloro 1,3-benzodioxol-5-yl) méthyl) 3-(2-thiényl) 1H-pyrazole 5-carboxylique,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Produits de formule (I) telle que définie à la revendication 1 dont les noms suivent :
- acide 4-((6-chloro-1,3-benzodioxol-5-yl) méthyl)-1-(cyclohexylméthyl)-3-phényl-1H-pyrazole-5-carboxylique,
- acide 1-butyl-5-((6-chloro-1,3-benzodioxol-5-yl) méthyl)-3-((5-carboxypentyl) thio)-1H-pyrazole-4-carboxylique,
- acide 1-((3-carboxycyclohexyl) méthyl)-4-((6-chloro-1,3-benzodioxol-5-yl) méthyl)-3-phényl-1H-pyrazole-5-carboxylique,
- acide 4-((6-chloro-1,3-benzodioxol-5-yl) méthyl)-1-cyclohexylméthyl)-3-(2-thiényl)-1H-pyrazole-5-carboxylique,
- acide 4-((6-chloro-1,3-benzodioxol-5-yl) méthyl)-1-(cyclohexylméthyl)-3-(2-pyridinyl)-1H-pyrazole-5-carboxylique,
- acide 4-((6-chloro-1,3-benzodioxol-5-yl) méthyl)-1-(cyclohexylméthyl)-3-(3-thiényl)-1H-pyrazole-5-carboxylique,
- acide 1-butyl 3-((4-(carboxyméthyl) cyclohexyl) thio) 5-((6(chloro 1,3-benzodioxol-5-yl) méthyl) 1H-pyrazole 4-carboxylique,
- acide 3-((4-(carboxyméthyl) cyclohexyl) thio) 5-((6-chloro 1,3-benzodioxol-5-yl) méthyl) 1-propyl 1H-pyrazole 4-carboxylique,
- acide 1-((bicyclo(2.2.2)oct-2-yl) méthyl) 4-((6-chloro 1,3-benzodioxol-5-yl) méthyl) 3-phényl 1H-pyrazole 5-carboxylique,
- acide 1-((4-carboxycyclohexyl) méthyl) 4-((6-chloro 1,3-benzodioxol-5-yl) méthyl) 3-(2-thiényl) 1H-pyrazole 5-carboxylique,
- acide cis/trans 1-((4-(carboxyméthyl) cyclohexyl) méthyl) 4-((6-chloro 1,3-benzodioxol-5-yl) méthyl) 3-(2-thiényl) 1H-pyrazole 5-carboxylique,
- acide 1-((2-(carboxyméthoxy) phényl) méthyl) 4-((6-chloro 1,3-benzodioxol-5-yl) méthyl) 3-(2-thiényl) 1H-pyrazole 5-carboxylique,
- acide 1-((3-carboxycyclohexyl) méthyl) 4-((6-chloro 1,3-benzodioxol-5-yl) méthyl) 3-(2-thiényl) 1H-pyrazole 5-carboxylique.

3. Procédé de préparation des produits de formule (I) telle que définie à la revendication 1, **caractérisé en ce que** :
ou bien l'on fait agir un produit de formule (II) :
ZCA'-CO-CH₂-CO-CO₂alk (II)
dans laquelle ZCA' représente un radical phényle, thiényle ou pyridyle, et alk représente un radical alkyle linéaire ou ramifié, renfermant au plus 4 atomes de carbone, avec le produit de formule (III) : 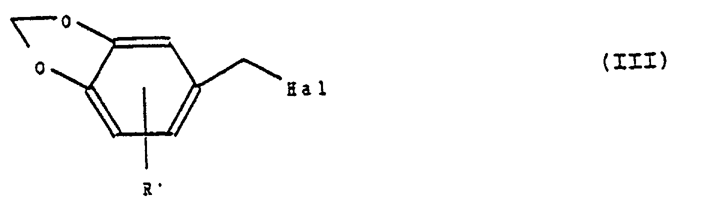 dans laquelle R' a la signification indiquée à la revendication 1 pour R, et Hal représente un atome d'halogène, pour obtenir le produit de formule (IV) : 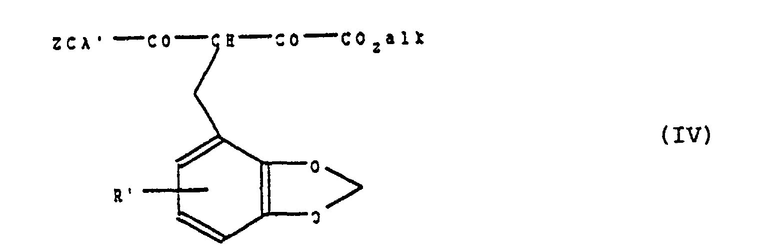 dans laquelle R', ZCA' et alk ont les significations indiquées ci-dessus, que l'on fait réagir avec l'hydrazine NH₂-NH₂ pour obtenir un produit de formule (V) : 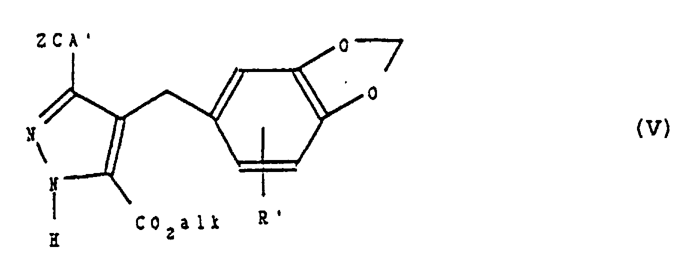 dans laquelle R', ZCA' et alk ont les significations indiquées ci-dessus,
que l'on fait réagir avec le composé de formule (VI) :
ZNB'-W (VI)
dans laquelle ZNB' représente un radical alkyle renfermant au plus 4 atomes de carbone lui-même substitué par un radical cyclohexyle, lui-même éventuellement substitué par un radical carboxy ou par un radical carboxyméthoxyméthyle, dans lequel les éventuelles fonctions réactives sont éventuellement protégées et W représente un atome d'halogène ou un radical tosyle, pour obtenir le produit de formule (I₁) 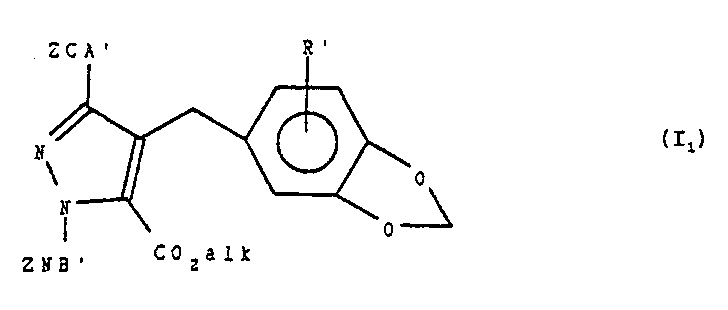 ou bien l'on fait agir un produit de formule (VII) : 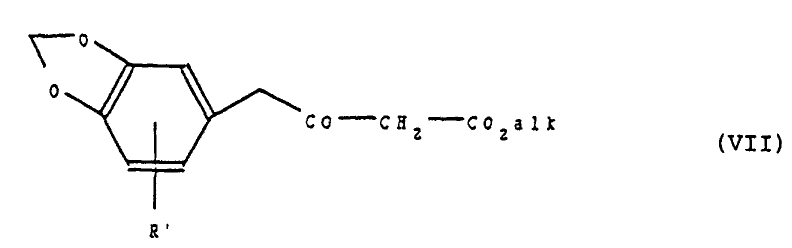 dans laquelle R' et alk ont les significations indiquées ci-dessus,
avec du disulfure de carbone et un produit de formule (VIII) :
R_{d}-Hal (VIII)
dans laquelle R_{d} représente un radical alkyle renfermant au plus 6 atomes de carbone éventuellement substitué par un radical carboxy ou par un radical phényle lui-même substitué par un radical alcoxy renfermant au plus 4 atomes de carbone, et Hal représente un atome d'halogène,
pour obtenir le produit de formule (IX) : 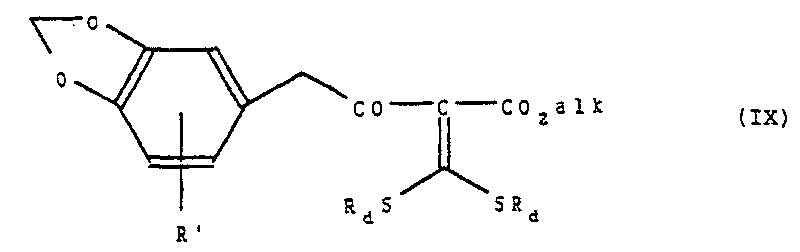 dans laquelle R', alk et R_{d} ont les significations indiquées ci-dessus et S représente un atome de soufre, que l'on traite avec un produit de formule (X) :
ZNA'-NH-NH₂ (X)
dans laquelle ZNA' représente un radical alkyle linéaire ou ramifié, renfermant au plus 4 atomes de carbone,pour obtenir le produit de formule (I₂) : 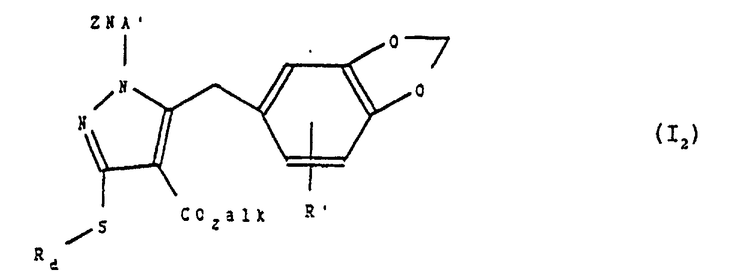 dans laquelle ZNA', alk, R_{d} et S ont les significations indiquées ci-dessus,
ou bien l'on fait agir un produit de formule (XI) : 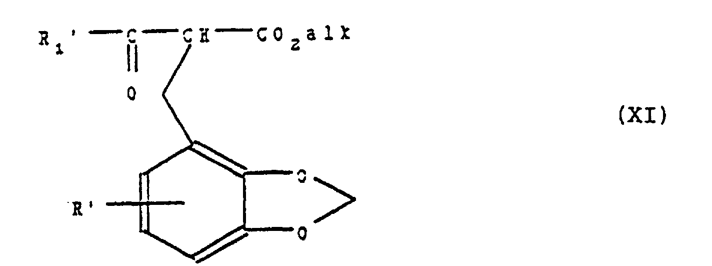 dans laquelle R' et alk ont les significations indiquées ci-dessus et R'₁ a la signification indiquée à la revendication 1 pour R₁, dans laquelle les éventuelles fonctions réactives sont éventuellement protégées, avec le dérivé de l'hydrazine suivant :
N≡C-(CH₂)₂-NH-NH₂
pour obtenir un produit de formule (XII) : 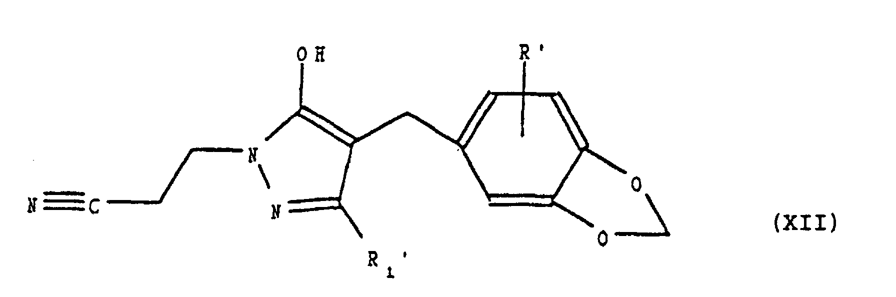 dans laquelle R' et R'₁ ont les significations indiquées ci-dessus, que l'on transforme en produit de formule (XIII) : 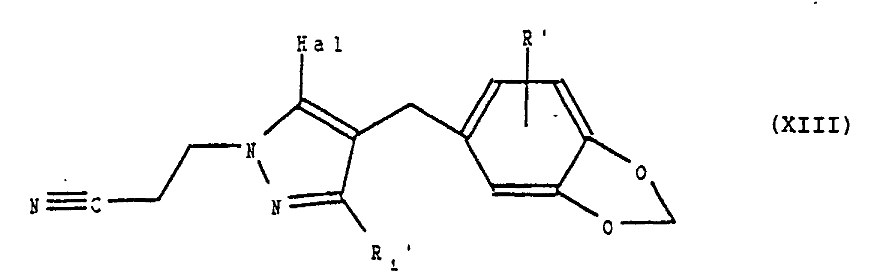 dans laquelle Hal, R' et R'₁ ont les significations indiquées ci-dessus, que l'on soumet à l'action dune base puis au composé de formule (VI) telle que définie ci-dessus,
pour obtenir le produit de formule (XIV) : 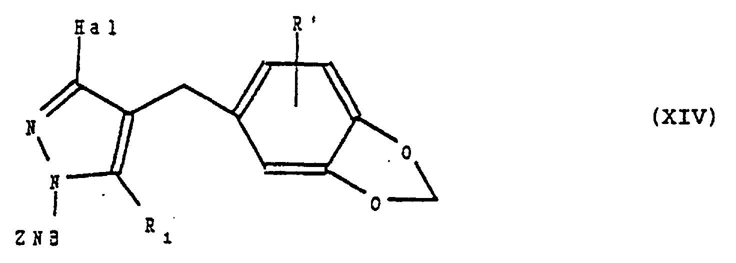 dans laquelle R', R'₁, ZNB' et Hal ont les significations indiquées ci-dessus,
que l'on soumet à une réaction de substitution par un dérivé de l'acide boronique ou par un organo metallique, sur l'atome d'halogéne pour obtenir le produit de formule (I₃) : 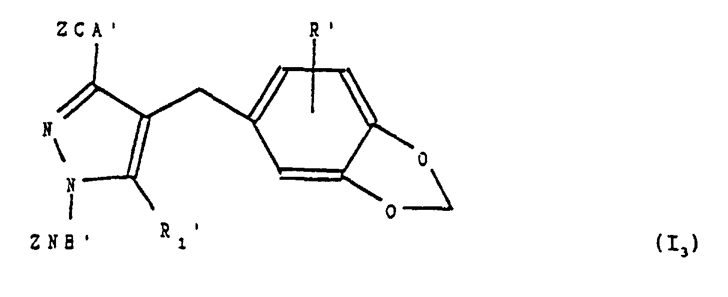 dans laquelle ZCA', ZNB', R' et R'₁ ont les significations indiquées ci-dessus,
ou bien l'on fait agir le produit de formule (VII) telle que définie ci-dessus, avec un produit de formule (XV) : 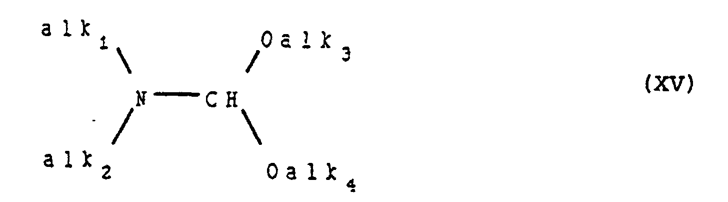 dans laquelle alk₁, alk₂, alk₃ et alk₄, identiques ou différents, représentent un radical alkyle linéaire ou ramifié, renfermant au plus 4 atomes de carbone, pour obtenir un produit de formule (XVI) : 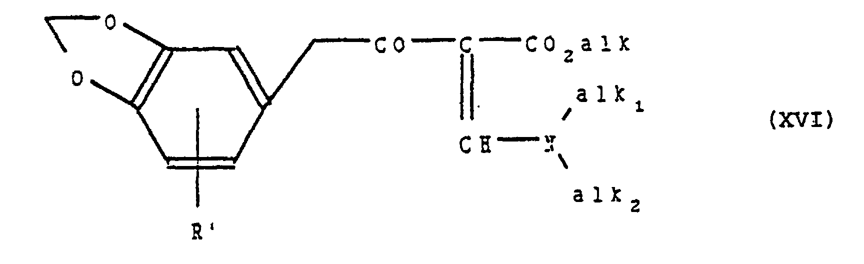 dans laquelle R', alk, alk₁ et alk₂ ont les significations indiquées ci-dessus, que l'on traite par l'hydrazine, pour obtenir le produit de formule (XVII) : 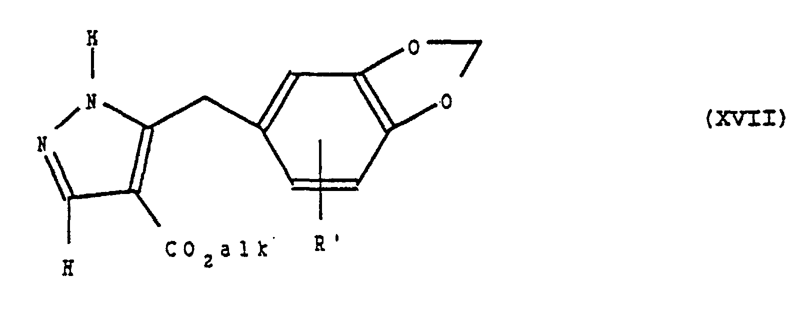 dans laquelle R' et alk ont les significations indiquées ci-dessus, que l'on traite par un agent d'halogénation pour obtenir un produit de formule (XVIII) : 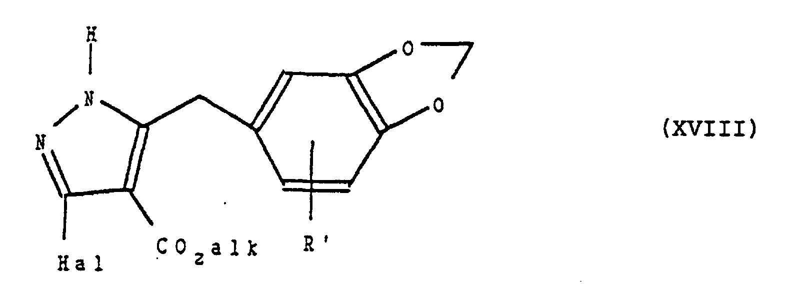 dans laquelle R', Hal et alk ont les significations indiquées ci-dessus, que l'on traite par le produit de formule (XIX) :
ZNA'-Hal (XIX)
dans laquelle ZNA' et Hal ont les significations indiquées ci-dessus, pour obtenir le produit de formule (XX) : 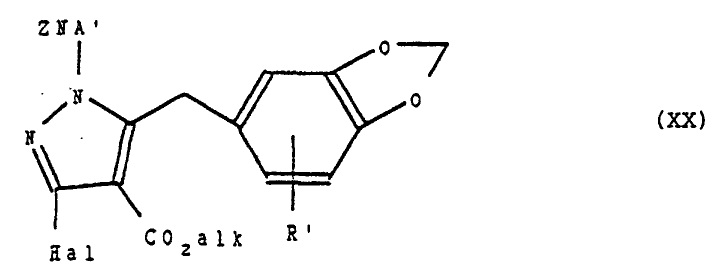 dans laquelle R' et alk ont les significations indiquées ci-dessus, sur lesquelles on fait agir un produit de formule (XXI) :
ZCB'-M (XXI)
dans laquelle ZCB' représente un radical alkylthio, renfermant au plus 6 atomes de carbone ou un radical cyclohexylthio, ces radicaux étant éventuellement substitués par un radical carboxy ou par un radical phényle lui-même substitué par un radical alcoxy renfermant au plus 4 atomes de carbone, et M représente un métal, pour obtenir le produit de formule (I₄) : 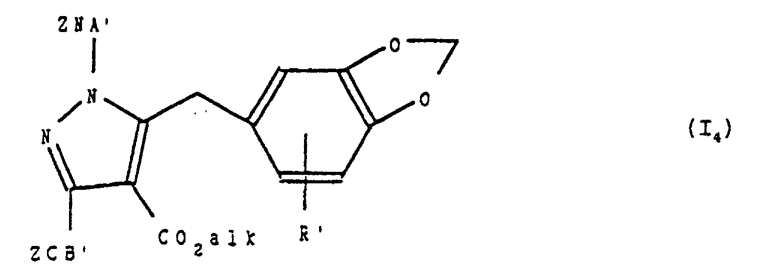 dans laquelle R', alk, ZNA' et ZCB' ont les significations indiquées ci-dessus,
produits de formules (I₁), (I₂), (I₃) et (I₄) telles que définies ci-dessus, que l'on peut soumettre, si désiré et si nécessaire, à l'une ou plusieurs des réactions de transformations suivantes, dans un ordre quelconque :
a) une réaction de saponification dé fonction ester en fonction acide,
f) une réaction de transformation de la fonction ester en radical hydroxyalkyle ou alcoxyalkyle puis en radical alkyle,
g) une réaction d'élimination des groupements protecteurs que peuvent porter les fonctions réactives protégées,
h) une réaction de salification par un acide minéral ou organique ou par une base pour obtenir le sel correspondant.
i) une réaction de dédoublement des formes racémiques en produits dédoublés,
lesdits produits de formule (I) ainsi obtenus étant sous toutes les formes isomères possibles racémiques, énantiomères et diastéréoisomères.

4. A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

5. A titre de médicaments, les produits de formule (I) tels que définis à la revendication 2 , ainsi que les sels d'addition avec les acides minéraux et organiques ou avec les bases minérales et organiques pharmaceutiquement acceptables desdits produits de formule (I).

6. Les compositions pharmaceutiques contenant à titre de principe actif, l'un au moins des médicaments tels que définis à Tune guelconque des revendications 4 et 5.

7. A titre de produits industriels, les composés tels que définis à la revendication 3 de formules (XII), (XIII) et (XIV), quand R'₁ est différent de carboxy, ainsi que les produits de formule (XX).

8. Utilisation des produits de formule (I) tels que définis aux revendications 1 et 2, pour la préparation de compositions pharmaceutiques destinées au traitement d'affections résultant d'une stimulation anormale des récepteurs de l'endothéline.

## Claims

1. Products of formula (I): 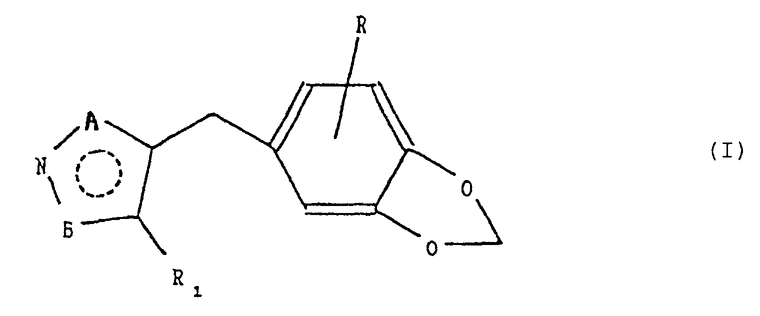 in which
A represents either a nitrogen atom substituted by a linear or branched alkyl radical, containing at most 4 carbon atoms, or a methine radical substituted by a phenyl, thienyl or pyridyl radical,
B represents either a nitrogen atom substituted by an alkyl radical containing at most 4 carbon atoms itself substituted by a cyclohexyl radical, itself optionally substituted by a carboxy radical or by a carboxymethoxymethyl radical,
or a methine radical substituted by an alkylthio radical, containing at most 6 carbon atoms or by a cyclohexylthio radical, these radicals being optionally substituted by a carboxy radical or by a phenyl radical itself substituted by an alkoxy radical containing at most 4 carbon atoms, it being understood that one of A and B represents a nitrogen atom,
and the other of A and B represents a methine radical, these radicals being substituted as indicated above,
R₁ represents a carboxy radical free or esterified by a linear or branched alkyl radical, containing at most 4 carbon atoms,
R represents a halogen atom,
as well as the following products:
- 1-butyl 3-((4-(carboxymethyl) cyclohexyl) thio) 5-((6(chloro 1,3-benzodioxol-5-yl) methyl) 1H-pyrazol 4-carboxylic acid,
- 3-((4-(carboxymethyl) cyclohexyl) thio) 5-((6-chloro 1,3-benzodioxol-5-yl) methyl) 1-propyl 1H-pyrazole 4-carboxylic acid,
- 1-((bicyclo(2.2.2)oct-2-yl) methyl) 4-((6-chloro 1,3-benzodioxol-5-yl) methyl) 3-phenyl 1H-pyrazole 5-carboxylic acid,
- cis/trans 1-((4-(carboxymethyl)cyclohexyl)methyl) 4-((6-chloro 1,3-benzodioxol-5-yl)methyl) 3-(2-thienyl) 1H-pyrazol 5-carboxylic acid,
- 1-((2-(carboxymethoxy) phenyl) methyl) 4-((6-chloro 1,3-benzodioxol-5-yl) methyl) 3-(2-thienyl) 1H-pyrazole 5-carboxylic acid,
said products of formula (I) being in all possible racemic, enantiomeric and diastereoisomeric isomer forms, as well as the addition salts with mineral and organic acids or with mineral and organic bases of said products of formula (I).

2. Products of formula (I) as defined in claim 1 the names of which follow:
- 4-((6-chloro-1,3-benzodioxol-5-yl) methyl)-1-(cyclohexylmethyl)-3-phenyl-1H-pyrazole-5-carboxylic acid,
- 1-butyl-5-((6-chloro-1,3-benzodioxol-5-yl) methyl)-3-((5-carboxypentyl) thio)-1H-pyrazole-4-carboxylic acid,
- 1-((3-carboxycyclohexyl) methyl)-4-((6-chloro-1,3-benzodioxol-5-yl) methyl)-3-phenyl-1H-pyrazole-5-carboxylic acid,
- 4-((6-chloro-1,3-benzodioxol-5-yl) methyl)-1-cyclohexylmethyl)-3-(2-thienyl)-1H-pyrazole-5-carboxylic acid,
- 4-((6-chloro-1,3-benzodioxol-5-yl) methyl)-1-(cyclohexylmethyl)-3-(2-pyridinyl)-1H-pyrazole-5-carboxylic acid,
- 4-((6-chloro-1,3-benzodioxol-5-yl) methyl)-1-(cyclohexylmethyl)-3-(3-thienyl)-1H-pyrazole-5-carboxylic acid,
- 1-butyl 3-((4-(carboxymethyl) cyclohexyl) thio) 5-((6(chloro 1,3-benzodioxol-5-yl) methyl) 1H-pyrazole 4-carboxylic acid,
- 3-((4-(carboxymethyl) cyclohexyl) thio) 5-((6-chloro 1,3-benzodioxol-5-yl) methyl) 1-propyl 1H-pyrazole 4-carboxylic acid,
- 1-((bicyclo(2.2.2)oct-2-yl) methyl) 4-((6-chloro 1,3-benzodioxol-5-yl) methyl) 3-phenyl 1H-pyrazol 5-carboxylic acid,
- 1-((4-carboxycyclohexyl) methyl) 9-((6-chloro 1,3-benzodioxol-5-yl) methyl) 3-(2-thienyl) 1H-pyrazole 5-carboxylic acid,
- cis/trans 1-((4-(carboxymethyl) cyclohexyl) methyl) 4-((6-chloro 1,3-benzodioxol-5-yl) methyl) 3-(2-thienyl) 1H-pyrazole 5-carboxylic acid,
- 1-((2-(carboxymethoxy) phenyl) methyl) 4-((6-chloro 1,3-benzodioxol-5-yl) methyl) 3-(2-thienyl) 1H-pyrazole 5-carboxylic acid,
- 1-((3-carboxycyclohexyl) methyl) 4-((6-chloro 1,3-benzodioxol-5-yl) methyl) 3-(2-thienyl) 1H-pyrazole 5-carboxylic acid.

3. Process for the preparation of the products of formula (I) as defined in claim 1, **characterized in that**:
either a product of formula (II):
ZCA'-CO-CH₂-CO-CO₂alk (II)
in which ZCA' represents a phenyl, thienyl or pyridyl radical, and alk represents a linear or branched alkyl radical, containing at most 4 carbon atoms, is reacted with the product of formula (III): 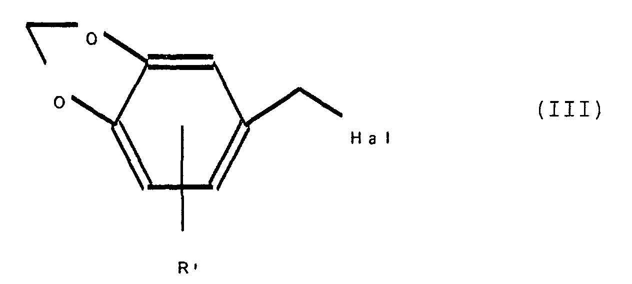 in which R' has the meaning indicated in claim 1 for R, and Hal represents a halogen atom, in order to obtain the product of formula (IV): 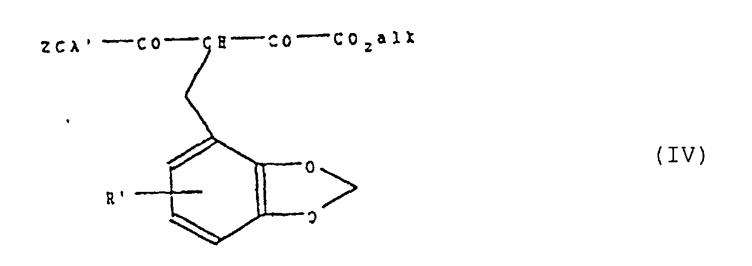 in which R', ZCA' and alk have the meanings indicated above, which is reacted with hydrazine NH₂-NH₂ in order to obtain a product of formula (V): 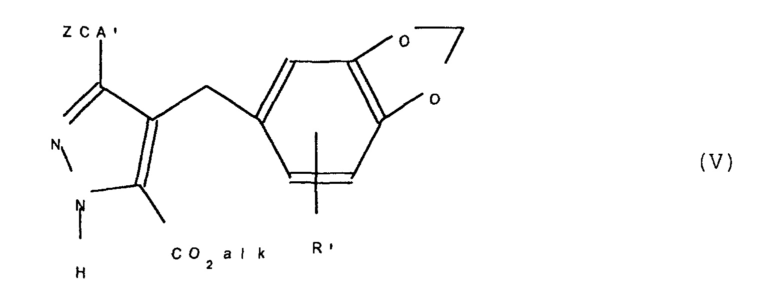 in which R', ZCA' and alk have the meanings indicated above, which is reacted with the compound of formula (VI):
ZNB'-W (VI)
in which ZNB' represents an alkyl radical containing at most 4 carbon atoms itself substituted by a cyclohexyl radical, itself optionally substituted by a carboxy radical or by a carboxymethoxymethyl radical, in which the optional reactive functions are optionally protected and W represents a halogen atom or a tosyl radical, in order to obtain the product of formula (I₁) : 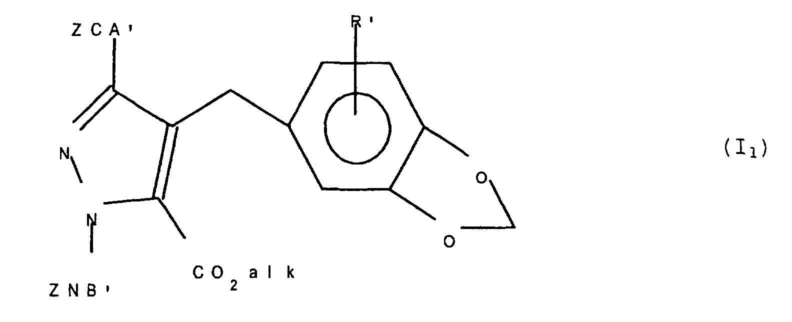 or a product of formula (VII) : 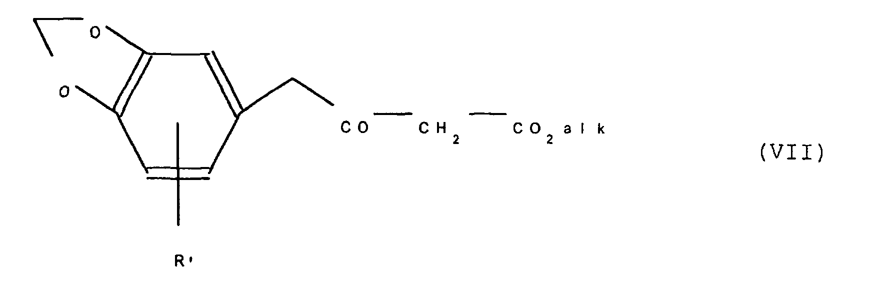 in which R' and alk have the meanings indicated above, is reacted with carbon disulphide and a product of formula (VIII):
R_{d}-Hal (VIII)
in which R_{d} represents an alkyl radical containing at most 6 carbon atoms optionally substituted by a carboxy radical or by a phenyl radical itself substituted by an alkoxy radical containing at most 4 carbon atoms, and Hal represents a halogen atom,
in order to obtain the product of formula (IX): 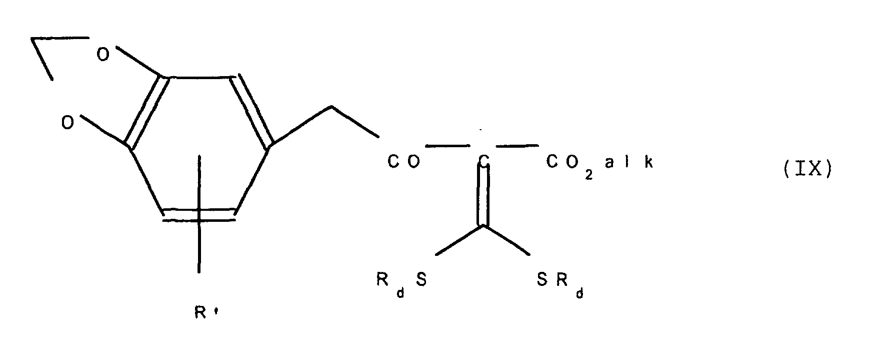 in which R', alk and R_{d} have the meanings indicated above and S represents a sulphur atom, which is treated with a product of formula (X):
ZNA'-NH-NH₂ (X)
in which ZNA' represents a linear or branched alkyl radical, containing at most 4 carbon atoms, in order to obtain the product of formula (I₂) : 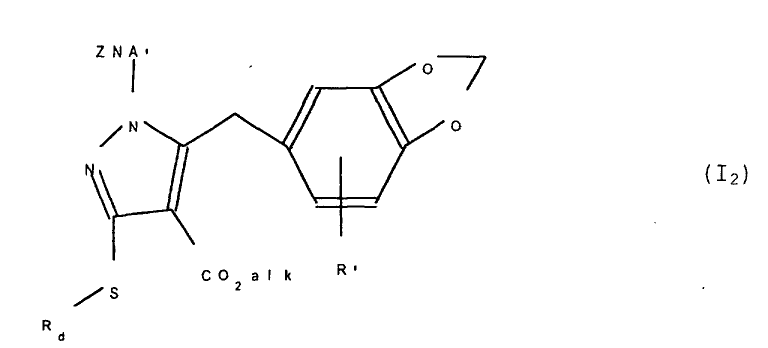 in which ZNA', alk, R_{d} and S have the meanings indicated above,
or a product of formula (XI): 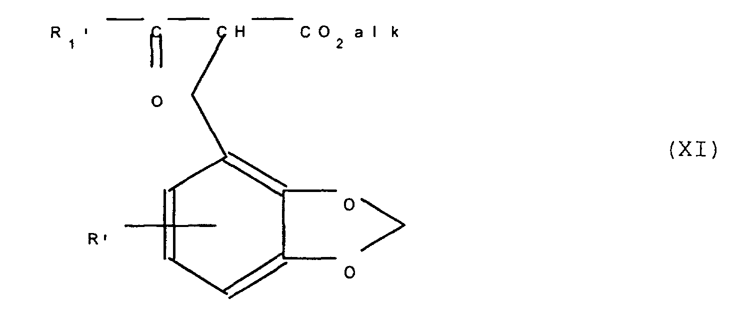 in which R' and alk have the meanings indicated above and R'₁ has the meaning indicated in claim 1 for R₁, in which the optional reactive functions are optionally protected, is reacted with the following hydrazine derivative:
N≡C-(CH₂)₂-NH-NH₂
in order to obtain a product of formula (XII): 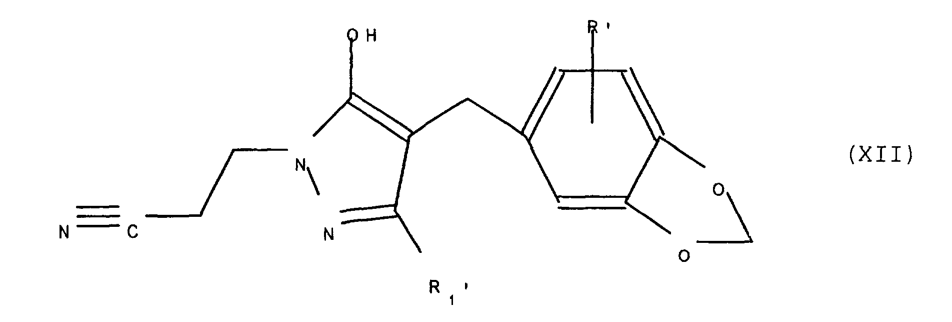 in which R' and R'₁ have the meanings indicated above, which is converted to a product of formula (XIII): 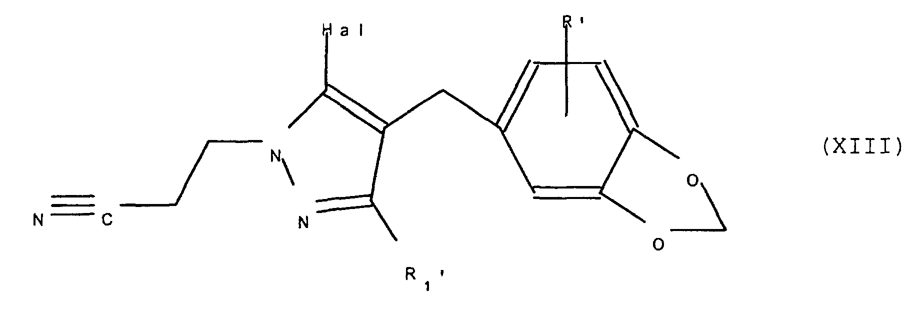 in which Hal, R' and R'₁ have the meanings indicated above, which is subjected to the action of a base then to the compound of formula (VI) as defined above,
in order to obtain the product of formula (XIV): 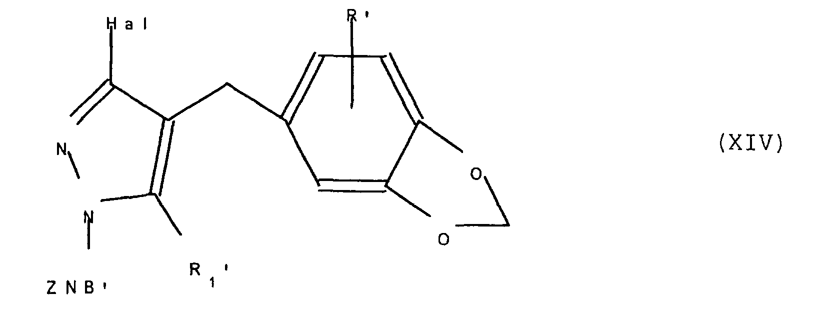 in which R', R'₁, ZNB' and Hal have the meanings indicated above,
which is subjected to a substitution reaction by a derivative of boronic acid or by an organo metallic, on the halogen atom in order to obtain the product of formula (I₃) : 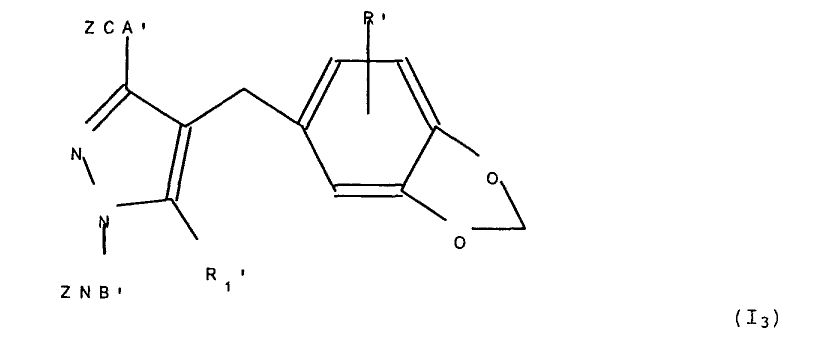 in which ZCA', ZNB', R' and R'₁ have the meanings indicated above,
or the product of formula (VII) as defined above, is reacted with a product of formula (XV): 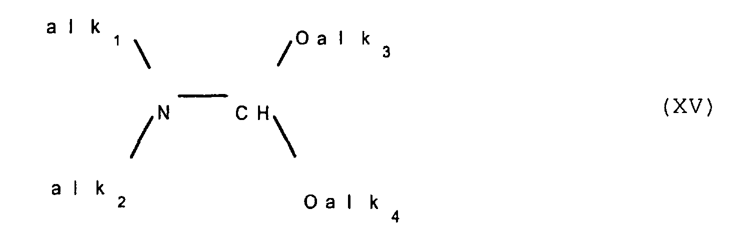 in which alk₁, alk₂, alk₃ and alk₄, identical or different, represent a linear or branched alkyl radical, containing at most 4 carbon atoms, in order to obtain a product of formula (XVI): 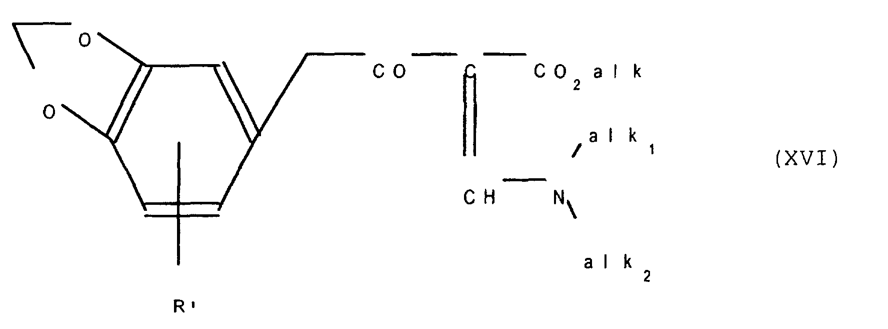 in which R', alk, alk₁ and alk₂ have the meanings indicated above, which is treated by hydrazine, in order to obtain the product of formula (XVII): 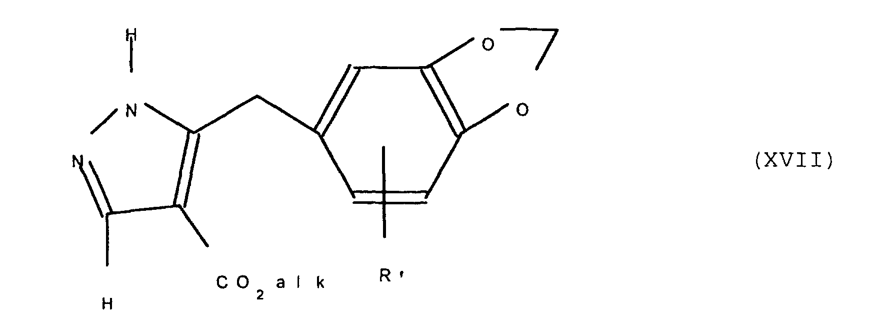 in which R' and alk have the meanings indicated above, which is treated with a halogenation agent in order to obtain a product of formula (XVIII): 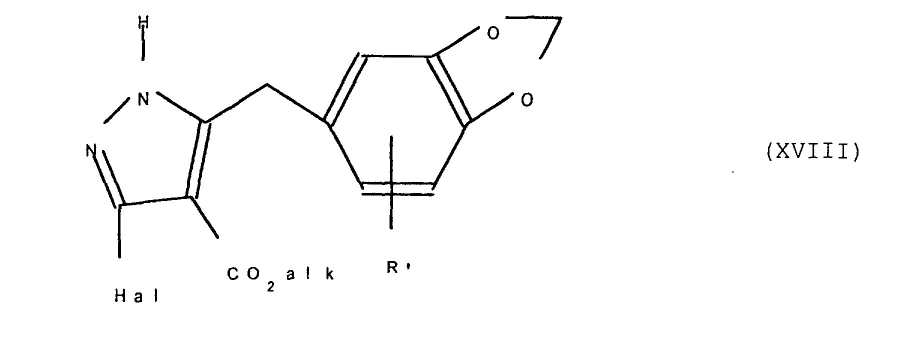 in which R', Hal and alk have the meanings indicated above, which is treated with the product of formula (XIX) :
ZNA'-Hal (XIX)
in which ZNA' and Hal have the meanings indicated above, in order to obtain the product of formula (XX): 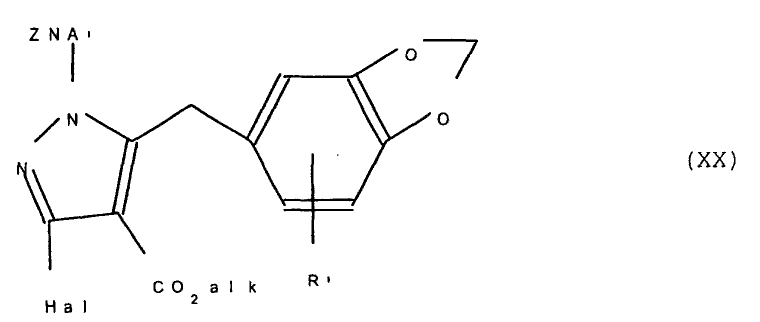 in which R' and alk have the meanings indicated above, on which a product of formula (XXI) is reacted:
ZCB'-M (XXI)
in which ZCB' represents an alkylthio radical, containing at most 6 carbon atoms or a cyclohexylthio radical, these radicals being optionally substituted by a carboxy radical or by a phenyl radical itself substituted by an alkoxy radical containing at most 4 carbon atoms, and M represents a metal, in order to obtain the product of formula (I₄) : 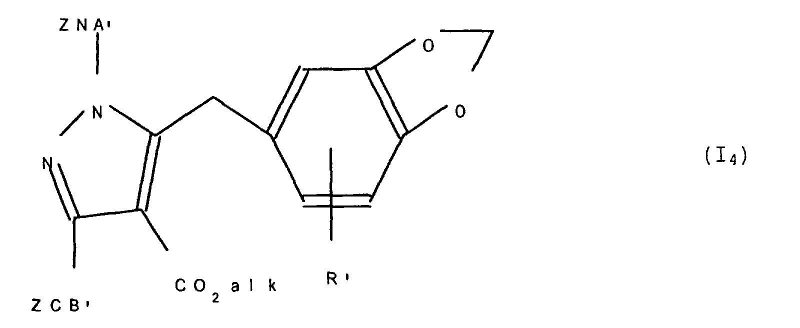 in which R', alk, ZNA' and ZCB' have the meanings indicated above,
which products of formulae (I₁), (I₂), (I₃) and (I₄) as defined above, can be subjected, if required and if necessary, to one or more of the following conversion reactions, in any order:
a) a saponification reaction of the ester function to an acid function,
f) a conversion reaction of the ester function to a hydroxyalkyl or alkoxyalkyl radical then to an alkyl radical,
g) an elimination reaction of the protective groups which can be carried by the protected reactive functions,
h) a salification reaction by a mineral or organic acid or by a base in order to obtain the corresponding salt,
i) a resolution reaction of the racemic forms into resolved products,
said products of formula (I) thus obtained being in all possible, racemic, enantiomeric and diastereoisomeric isomer forms.

4. As medicaments, the products as defined by formula (I) of claim 1, said products of formula (I) being in all possible, racemic, enantiomeric and diastereoisomeric isomer forms, as well as the addition salts with pharmaceutically acceptable mineral and organic acids or with mineral and organic bases of said products of formula (I).

5. As medicaments, the products of formula (I) as defined in claim 2, as well as the addition salts with pharmaceutically acceptable mineral and organic acids or with mineral and organic bases of said products of formula (I).

6. Pharmaceutical compositions containing as active ingredient, at least one of the medicaments as defined in any one of claims 4 and 5.

7. As industrial products, the compounds as defined in claim 3 of formulae (XII), (XIII) and (XIV), when R'₁ is different from carboxy, as well as the products of formula (XX).

8. Use of the products of formula (I) as defined in claims 1 and 2, for the preparation of pharmaceutical compositions intended for the treatment of illnesses resulting from an abnormal stimulation of the endothelin receptors.

## Patentansprüche

1. Produkte der Formel (I): 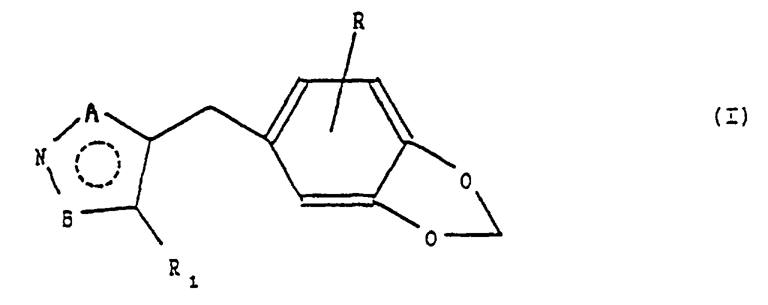 worir
A entweder ein Stickstoffatom, das mit einer geradkettigen oder verzweigten Alkylgruppe mit höchstens 4 Kohlenstoffatomen substituiert ist, oder
eine Methingruppe, die mit einer Phenyl-, Thienyl- oder Pyridyl-Gruppe substituiert ist, darstellt;
B entweder ein Stickstoffatom, das mit einer Alkylgruppe mit höchstens 4 Kohlenstoffatomen substituiert ist, die selbst mit einer Cyclohexylgruppe substituiert ist, welche gegebenenfalls selbst mit einer Carboxygruppe oder mit einer Carboxymethoxymethyl-Gruppe substituiert ist, oder
eine Methingruppe, die mit einer Alkylthiogruppe mit höchstens 6 Kohlenstoffatomen oder mit einer Cyclohexylthiogruppe substituiert ist, wobei diese Gruppen gegebenenfalls mit einer Carboxygruppe oder mit einer Phenylgruppe substituiert sind, welche selbst mit einer Alkoxygruppe mit höchstens 4 Kohlenstoffatomen substituiert ist, darstellt, wobei eine der Gruppen A und B ein Stickstoffatom darstellt und die andere der Gruppen A und B eine Methingruppe darstellt und diese Gruppen wie oben angegeben substituiert sind;
R₁ eine Carboxygruppe darstellt, die frei ist oder mit einer geradkettigen oder verzweigten Alkylgruppe mit höchstens 4 Kohlenstoffatomen verestert ist;
R ein Halogenatom darstellt;
sowie die folgenden Produkte:
- 1-Butyl-3-((4-(carboxymethyl)cyclohexyl)thio)-5-((6(chlor-1,3-benzodioxol-5-yl)methyl)-1H-pyrazol-4-carbonsäure,
- 3-((4-(Carboxymethyl)cyclohexyl)thio)-5-((6-chlor-1,3-benzodioxol-5-yl)methyl)-1-propyl-1H-pyrazol-4-carbonsäure,
- 1-((Bicyclo(2.2.2)oct-2-yl)methyl)-4-((6-chlor-1,3-benzodioxol-5-yl)methyl)-3-phenyl-1H-pyrazol-5-carbonsäure,
- cis/trans-1-((4-(Carboxymethyl)cyclohexyl)methyl)-4-((6-chlor-1,3-benzodioxol-5-yl)methyl)-3-(2-thienyl)-1H-pyrazol-5-carbonsäure,
- 1-((2-(Carboxymethoxy)phenyl)methyl)-4-((6-chlor-1,3-benzodioxol-5-yl)methyl)-3-(2-thienyl)-1H-pyrazol-5-carbonsäure,
die Produkte der Formel (I), die in allen möglichen Isomerenformen wie als racemische, enantiomere und diastereomere Formen vorliegen können, sowie die Additionssalze der Produkte der Formel (I) mit Mineralsäuren und organischen Säuren oder mit Mineralbasen und organischen Basen.

2. Produkte der Formel (I), wie sie in Anspruch 1 definiert sind, mit den folgenden Namen:
- 4-((6-Chlor-1,3-benzodioxol-5-yl)methyl)-1-(cyclohexylmethyl)-3-phenyl-1H-pyrazol-5-carbonsäure,
- 1-Butyl-5-((6-chlor-1,3-benzodioxol-5-yl)methyl)-3-((5-carboxypentyl)thio)-1H-pyrazol-4-carbonsäure,
- 1-((3-Carboxycyclohexyl)methyl)-4-((6-chlor-1,3-benzodioxol-5-yl)methyl)-3-phenyl-1H-pyrazol-5-carbonsäure,
- 4-((6-Chlor-1,3-benzodioxol-5-yl)methyl)-1-(cyclohexylmethyl)-3-(2-thienyl)-1H-pyrazol-5-carbonsäure,
- 4-((6-Chlor-1,3-benzodioxol-5-yl)methyl)-1-(cyclohexylmethyl)-3-(2-pyridinyl)-1H-pyrazol-5-carbonsäure,
- 4-((6-Chlor-1,3-benzodioxol-5-yl)methyl)-1-(cyclohexylmethyl)-3-(3-thienyl)-1H-pyrazol-5-carbonsäure,
- 1-Butyl-3-((4-(carboxymethyl)cyclohexyl)thio)-5-((6(chlor-1,3-benzodioxol-5-yl)methyl)-1H-pyrazol-4-carbonsäure,
- 3-((4-(Carboxymethyl)cyclohexyl)thio)-5-((6-chlor-1,3-benzodioxol-5-yl)methyl)-1-propyl-1H-pyrazol-4-carbonsäure,
- 1-(Bicyclo(2.2.2)oct-2-yl)methyl)-4-((6-chlor-1,3-benzodioxol-5-yl)methyl)-3-phenyl-1H-pyrazol-5-carbonsäure,
- 1-((4-Carboxycyclohexyl)methyl)-4-((6-chlor-1,3-benzodioxol-5-yl)methyl)-3-(2-thienyl)-1H-pyrazol-5-carbonsäure,
- cis/trans-1-((4-(Carboxymethyl)cyclohexyl)methyl)-4-((6-chlor-1,3-benzodioxol-5-yl)methyl)-3-(2-thienyl)-1H-pyrazol-5-carbonsäure,
- 1-((2-(Carboxymethoxy)phenyl)methyl)-4-((6-chlor-1,3-benzodioxol-5-yl)methyl)-3-(2-thienyl)-1H-pyrazol-5-carbonsäure,
- 1-((3-Carboxycyclohexyl)methyl)-4-((6-chlor-1,3-benzodioxol-5-yl)methyl)-3-(2-thienyl)-1H-pyrazol-5-carbonsäure.

3. Verfahren zur Herstellung der Produkte der Formel (I), wie sie in Anspruch 1 definiert ist, **dadurch gekennzeichnet, dass** man entweder ein Produkt der Formel (II):
ZCA'-CO-CH₂-CO-CO₂alk (II)
worin ZCA' eine Phenyl-, Thienyl- oder Pyridyl-Gruppe darstellt, und alk eine geradkettige oder verzweigte Alkylgruppe mit höchstens 4 Kohlenstoffatomen darstellt, mit dem Produkt der Formel (III): 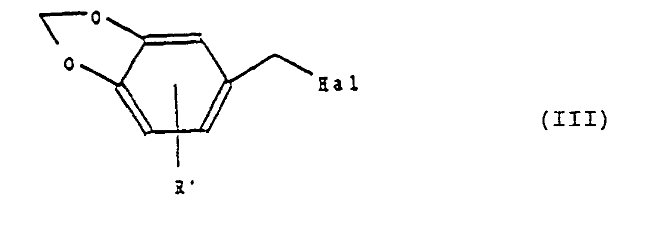 worin R' die in Anspruch 1 für R angegebene Bedeutung hat und Hal ein Halogenatom darstellt, reagieren lässt, wobei man das Produkt der Formel (IV) erhält: 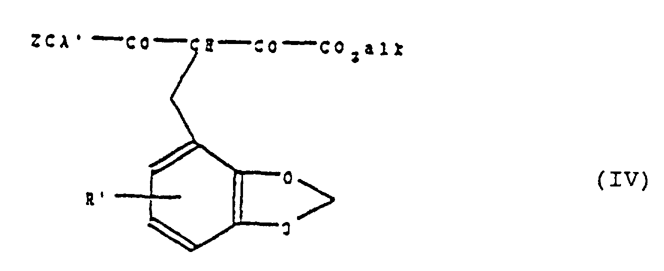 worin R', ZCA' und alk die oben gegebenen Bedeutungen haben, das man dann mit Hydrazin NH₂-NH₂ reagieren lässt, wobei ein Produkt der Formel (V) erhalten wird: 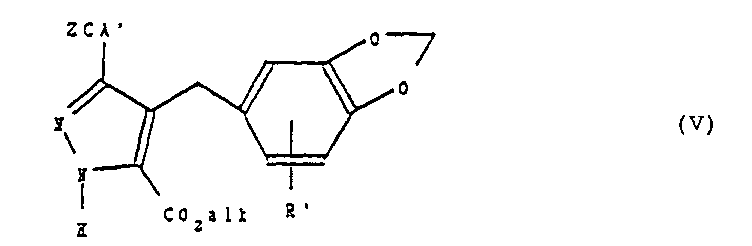 worin R', ZCA' und alk die oben gegebenen Bedeutungen haben, welches man mit der Verbindung der Formel (VI):
ZNB'-W (VI)
worin ZNB' eine Alkylgruppe mit höchstens 4 Kohlenstoffatomen darstellt, die selbst mit einer Cyclohexylgruppe substituiert ist, die gegebenenfalls selbst mit einer Carboxygruppe oder einer Carboxymethoxymethylgruppe substituiert ist, in der die eventuellen reaktiven Funktionen gegebenenfalls geschützt sind, und W ein Halogenatom oder eine Tosylgruppe darstellt, reagieren lässt, um das Produkt der Formel (I₁) zu erhalten: 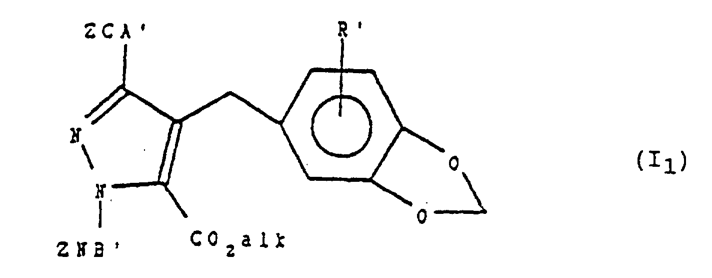 oder dass man ein Produkt der Formel (VII): 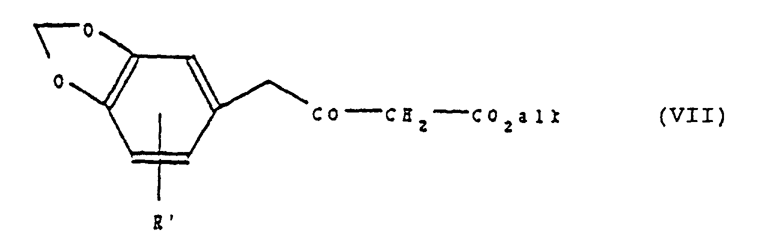 worin R' und alk die oben gegebenen Bedeutungen haben, mit Kohlenstoffdisulfid und einem Produkt der Formel (VIII):
R_{d}-Hal (VIII)
worin R_{d} eine Alkylgruppe mit höchstens 6 Kohlenstoffatomen darstellt, die gegebenenfalls mit einer Carboxygruppe oder mit einer Phenylgruppe substituiert ist, die selbst mit einer Alkoxygruppe mit höchstens 4 Kohlenstoffatomen substituiert ist, und Hal ein Halogenatom darstellt, reagieren lässt, um das Produkt der Formel (IX) zu erhalten: 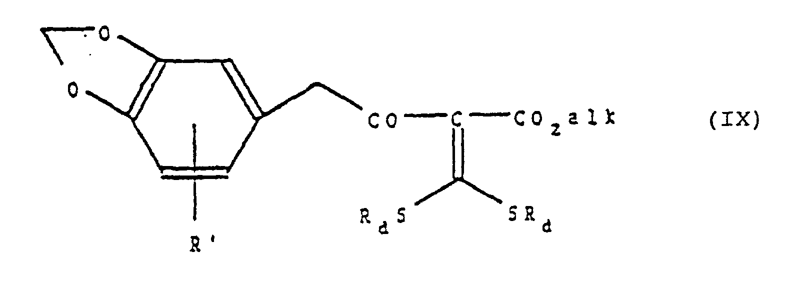 worin R', alk und R_{d} die oben gegebenen Bedeutungen haben und S ein Schwefelatom darstellt, das man mit einem Produkt der Formel (X):
ZNA'-NH-NH₂ (X)
worin ZNA' eine lineare oder verzweigte Alkylgruppe mit höchstens 4 Kohlenstoffatomen darstellt, behandelt, um das Produkt der Formel (I₂) zu erhalten: 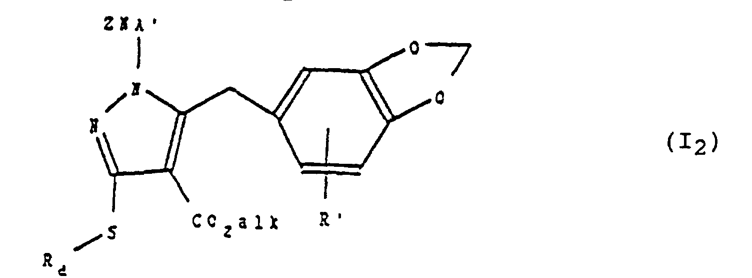 worin ZNA', alk, R_{d} und S die oben gegebenen Bedeutungen haben,
oder dass man ein Produkt der Formel (XI): 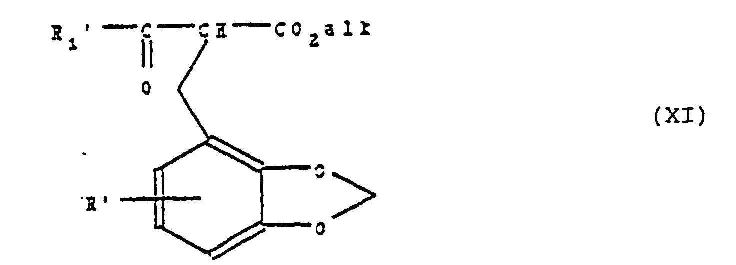 worin R' und alk die oben gegebenen Bedeutungen haben und R'₁ die in Anspruch 1 für R₁ gegebene Bedeutung hat, in der die eventuellen reaktiven Funktionen gegebenenfalls geschützt sind, mit dem folgenden Hydrazinderivat:
N≡C-(CH₂)₂-NH-NH₂
reagieren lässt, um ein Produkt der Formel (XII) zu erhalten: 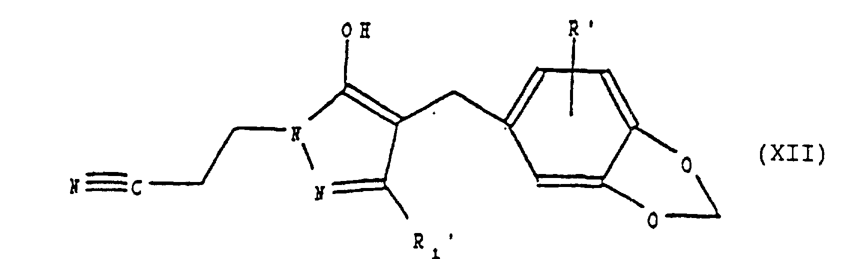 in der R' und R'₁ die oben gegebenen Bedeutungen haben, das man in ein Produkt der Formel (XIII) überführt: 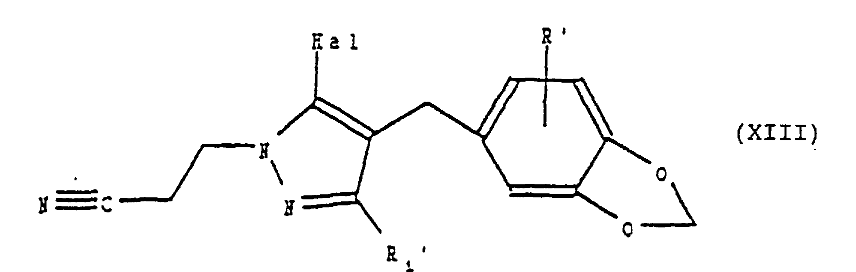 worin Hal, R' und R'₁ die oben gegebenen Bedeutungen haben, das man der Wirkung einer Base, dann der Verbindung der Formel (VI), wie sie oben definiert ist, unterwirft, um das Produkt der Formel (XIV) zu erhalten: 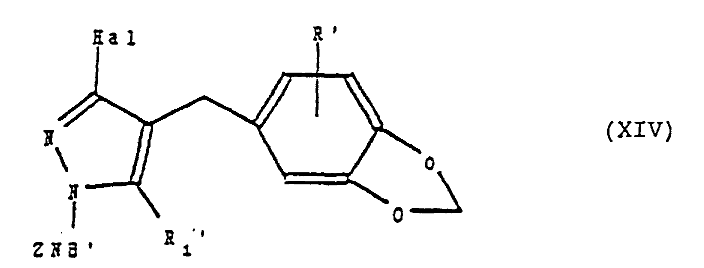 worin R', R'₁, ZNB' und Hal die oben gegebenen Bedeutungen haben, das man einer Substitutionsreaktion mit einem Derivat der Borsäure oder einen organometallischen Derivat am Halogenatom unterwirft, um das Produkt der Formel (I₃) zu erhalten: 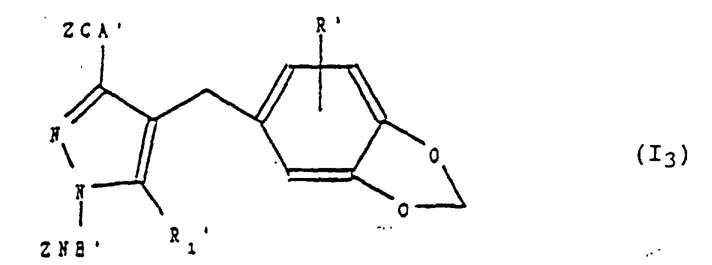 worin ZCA', ZNB', R' und R'₁ die oben gegebenen Bedeutungen haben,
oder dass man das Produkt der Formel (VII), wie sie oben definiert ist, mit einem Produkt der Formel (XV): 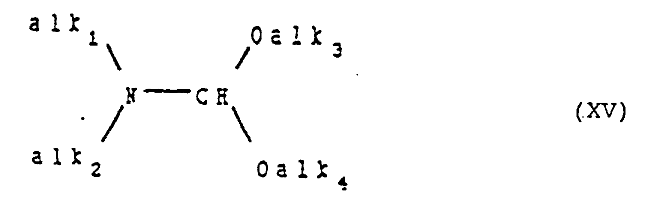 worin alk₁, alk₂, alk₃ und alk₄, die gleich oder unterschiedlich sind, eine lineare oder verzweigte Alkylgruppe mit höchstens 4 Kohlenstoffatomen darstellen, reagieren lässt, um ein Produkt der Formel (XVI) zu erhalten: 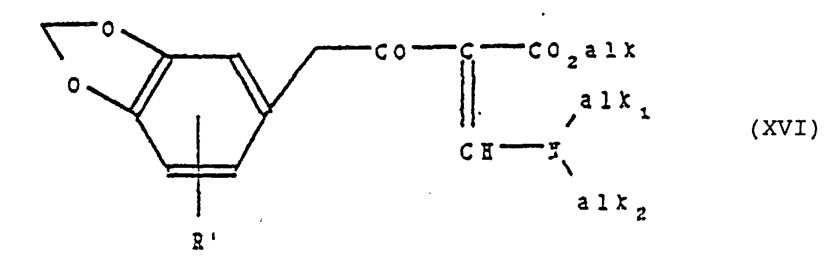 worin R', alk, alk₁ und alk₂ die oben gegebenen Bedeutungen haben, das man dann mit Hydrazin behandelt, um das Produkt der Formel (XVII) zu erhalten: 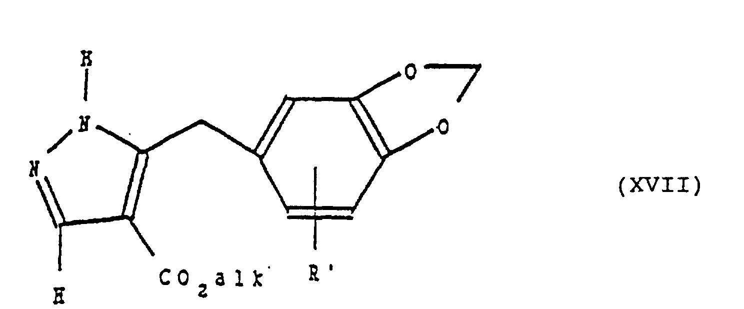 worin R' und alk die oben gegebenen Bedeutungen haben, das man mit einem Halogenierungsmittel behandelt, um ein Produkt der Formel (XVIII) zu erhalten: 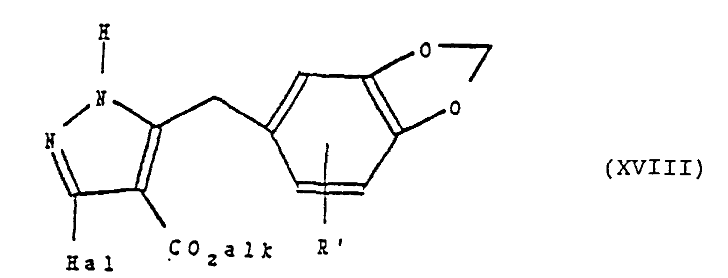 worin R', Hal und alk die oben gegebenen Bedeutungen haben, das man mit dem Produkt der Formel (XIX):
ZNA'-Hal (XIX)
worin ZNA' und Hal die oben gegebenen Bedeutungen besitzen, behandelt, um das Produkt der Formel (XX) zu erhalten: 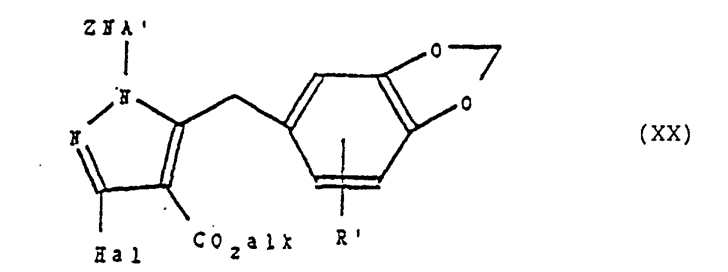 worin R' und alk die oben gegebenen Bedeutungen haben, worauf man ein Produkt der Formel (XXI):
ZCB'-M (XXI)
worin ZCB' eine Alkylthiogruppe mit höchstens 6 Kohlenstoffatomen oder eine Cyclohexylthiogruppe darstellt, wobei diese Gruppen gegebenenfalls mit einer Carboxygruppe oder einer Phenylgruppe substituiert sind, die selbst mit einer Alkoxygruppe mit höchstens 4 Kohlenstoffatomen substituiert ist, und M ein Metall darstellt, einwirken lässt, um das Produkt der Formel (I₄) zu erhalten: 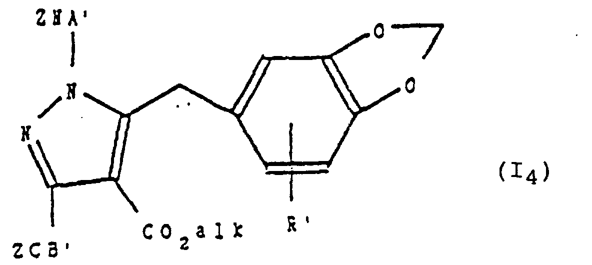 worin R', alk, ZNA' und ZCB' die oben gegebenen Bedeutungen besitzen,
wobei man die Produkte der Formeln (I₁), (I₂), (I₃) und (I₄), wie sie oben definiert sind, wenn gewünscht und wenn notwendig, einer oder mehreren der folgenden Reaktionen zur Umwandlung in beliebiger Reihenfolge unterwerfen kann:
a) eine Verseifungsreaktion der Esterfunktion zur Säurefunktion,
f) eine Umwandlungsreaktion der Esterfunktion in eine Hydroxyalkylgruppe oder eine Alkoxyalkylgruppe, danach in eine Alkylgruppe,
g) eine Eliminierungsreaktion für Schutzgruppen, die die geschützten reaktiven Funktionen tragen können,
h) eine Salzbildungsreaktion mit einer Mineralsäure oder einer organischen Säure oder einer Base, um das entsprechende Salz zu erhalten,
i) eine Reaktion zur Auftrennung der racemischen Formen in getrennte Formen,
wobei die so erhaltenen Produkte der Formel (I) in allen möglichen isomeren Formen vorliegen können, z. B. in racemischen, enantiomeren und diastereomeren Formen.

4. Die Produkte, wie sie durch die Formel (I) in Anspruch 1 definiert sind, wobei die Produkte der Formel (I) in allen möglichen isomeren Formen, in racemischen, enantiomeren und diastereomeren Formen, vorliegen können, sowie die Additionssalze der Produkte der Formel (I) mit Mineralsäuren und organischen Säuren oder mit Mineralbasen und organischen Basen, die pharmazeutisch annehmbar sind, als Arzneimittel.

5. Die Produkte der Formel (I), wie sie in Anspruch 2 definiert sind, sowie die Additionssalze der Produkte der Formel (I) mit Mineralsäuren und organischen Säuren oder mit Mineralbasen und organischen Basen, die pharmazeutisch annehmbar sind, als Arzneimittel.

6. Pharmazeutische Zusammensetzungen, die als Wirkstoff mindestens eines der Medikamente, wie sie in Anspruch 4 und Anspruch 5 definiert sind, enthalten.

7. Verbindungen nach Anspruch 3 der Formel (XII), (XIII) und (XIV), wenn R'₁ nicht Carboxy ist, sowie die Produkte der Formel (XX) als industrielle Produkte.

8. Verwendung der Produkte der Formel (I), wie sie in Anspruch 1 and Anspruch 2 definiert sind, zur Herstellung von pharmazeutischen Zusammensetzungen, die zur Behandlung von Krankheiten bestimmt sind, welche aus einer anormalen Stimulation der Endothelialrezeptoren resultieren.
